# EUROPEAN PATENT SPECIFICATION

(11) **EP 1 507 760 B1**
(45) Date of publication and mention of the grant of the patent: **01.08.2007**
(21) Application number: 03722311.2
(22) Date of filing: 15.05.2003
(51) Int. Cl.: C07D 213/02, A61K 31/4425, A61P 35/00

(54) **CYANOGUANIDINE PRODRUGS**
CYANOGUANIDIN PRODRUGS
PROMEDICAMENTS DE CYANOGUANIDINE

(30) Priority: 17.05.2002 US 380836 P
(43) Date of publication of application: 23.02.2005
(73) Proprietor: LEO PHARMA A/S, 2750 Ballerup (DK)
(72) Inventor: BINDERUP, Ernst, Torndal, DK-2630 Taastrup (DK); DUVOLD, Tore, DK-1871 Frederiksberg C (DK)
(74) Representative: Duckworth, Timothy John
(86) International application number: PCT/DK2003/000318
(87) International publication number: WO 2003/097601

(56) References cited:
- WO-A-00/61559
- WO-A-02/42265
- WO-A-98/54144

## Description

### FIELD OF INVENTION

The present invention relates to novel pyridyl cyanoguanidine prodrugs and their inclusion in pharmaceutical compositions, as well as their use in the manufacture of medicaments.

### BACKGROUND OF THE INVENTION

Pyridyl cyanoguanidines such as pinacidil (N-1,2,2-trimethylpropyl-N'-cyano-N"-(4-pyridyl)guanidine) were originally discovered to be potassium channel openers and were consequently developed as antihypertensive agents. Replacement of the side chain of pinacidil by longer aryl-containing side chains caused a loss of the antihypertensive activity, but such compounds were, on the other hand, found to show antitumour activity on oral administration in a rat model carrying Yoshida ascites tumours.

Different classes of pyridyl cyanoguanidines with antiproliferative activity are disclosed in, for instance, EP 660 823, WO 98/54141, WO 98/54143, WO 98/54144, WO 02/42265, WO 00/61559, WO 98/54145, WO 00/61559 and WO 00/61561. The structure-activity relationships (SAR) of such compounds are discussed in C. Schou et al., Bioorganic and Medicinal Chemistry Letters 7(24), 1997, pp. 3095-3100, in which the antiproliferative effect of a number of pyridyl cyanoguanidines was tested *in vitro* on different human lung and breast cancer cell lines as well as on normal human fibroblasts. The compounds were also tested *in vivo* in nude mice carrying a human lung cancer tumour xenograft. Based on the SAR analysis, a specific compound (N-(6-(4-chlorophenoxy)hexyl)-N'-cyano-N"-(4-pyridyl)guanidine) was selected for its high antiproliferative activity *in vitro* and potent antitumour activity in the nude mouse model.

P-J V Hjarnaa et al., Cancer Res. 59, 1999, pp. 5751-5757, report on the results of further testing of the compound N-(6-(4-chlorophenoxy)hexyl)-N'-cyano-N"-(4-pyridyl)guanidine in *in vitro* and *in vivo* tests. The compound exhibited a potency *in vitro* which was comparable to that of the reference cytostatic agents daunorubicin and paclitaxel, while showing considerably less antiproliferative activity on normal human endothelial cells. In *in vivo* tests using nude mice transplanted with human tumour cells, the compound showed substantial antitumour activity, also against tumour cells that were resistant to conventional anticancer drugs such as paclitaxel.

### SUMMARY OF THE INVENTION

While, as indicated above, pyridyl cyanoguanidines are promising antitumour agents with an extremely interesting activity profile, they are highly lipophilic and consequently sparingly soluble compounds and are, as such, generally available for oral administration only. However, many cancer patients are in a severely debilitated condition as a result of their illness giving rise to problems with patient compliance with respect to oral administration of drugs.

It is therefore an object of the present invention to provide pyridyl cyanoguanidines in the form of prodrugs with an improved solubility profile which prodrugs may be included in pharmaceutical compositions suitable for parenteral administration, i.e. liquid compositions in which the prodrug is dissolved in sufficient amounts to be converted to therapeutically effective quantities of the active compound on administration of the composition. The compounds of the present invention exhibit good solubility in water, even at pH values around physiological pH making them ideal candidates for parenteral administration.

Furthermore, it has been found that pyridyl cyanoguanidine prodrugs of the invention exhibit an improved gastrointestinal absorption on oral administration. Consequently, it is another object of the Invention to provide oral formulations of pyridyl cyanoguanidines as prodrugs with improved bioavailability.

Accordingly, the present invention relates to a compound of the general formula I 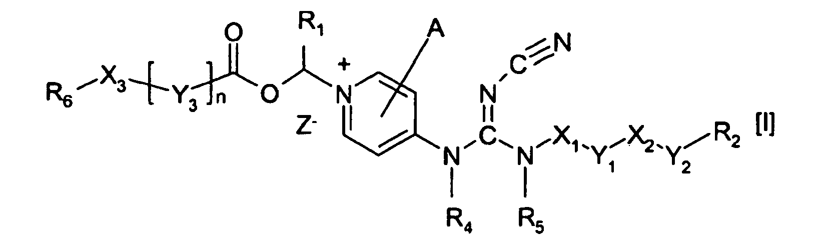 wherein X₁ is a straight, branched and/or cyclic hydrocarbon diradical, optionally substituted with one or more hydroxy, halogen, nitro, amino or cyano;
X₂ is a bond; a straight, branched and/or cyclic hydrocarbon diradical, optionally substituted with one or more hydroxy, halogen, nitro, amino, cyano, aminosulfonyl, alkylsulfonylamino, alkylcarbonyl, formyl, aminocarbonyl or alkylcarbonylamino; a heteroarylene or non-aromatic heterocyclic hydrocarbon diradical, all of which are optionally substituted with one or more straight, branched and/or cyclic non-aromatic hydrocarbon radical, hydroxyl, halogen, amino, nitro, cyano, aminosulfonyl, alkylsulfonylamino, alkylcarbonyl, formyl, aminocarbonyl or alkylcarbonylamino;
X₃ is a straight, branched and/or cyclic hydrocarbon diradical, optionally substituted with one or more substituents selected from the group consisting of hydroxy, halogen, nitro, amino, cyano, aminosulfonyl, alkylsulfonylamino, alkylcarbonyl, formyl, aminocarbonyl or alkylcarbonylamino; with the proviso that when n is 0, X₃ may also be a bond;
Y₁ is a bond, O, S, S(O), S(O)₂, C(O), NH-C(O) or C(O)-NH;
Y₂ is a bond, an ether diradical (R'-O-R"), an amine diradical (R'-N-R"), O, S, S(O), S(O)₂, C(O), NH-C(O), C(O)-NH, SO₂-N(R') or N(R')-SO₂ wherein R' and R" are independently straight or branched hydrocarbon diradicals containing up to 4 carbon atoms;
Y₃ is O;
R₁ is hydrogen or straight, branched and/or cyclic alkyl, optionally substituted with phenyl; or an aromatic hydrocarbon radical;
R₂ is hydrogen, or aryl or heteroaryl, both of which are optionally substituted with one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxy, cyano or nitro; tetrahydropyranyloxy, di-(C₁₋₄ alkoxy)phosphinoyloxy or C₁₋₄ alkoxycarbonylamino;
R₄ is hydrogen;
R₅ is hydrogen, or a straight, branched and/or cyclic hydrocarbon radical, optionally substituted with halogen, hydroxyl, halogen, amino, nitro or cyano;
R₆ is piperidyl attached to the 3 or 4 position to X₃;
A is hydrogen, an optionally substituted, straight, branched and/or cyclic hydrocarbon radical, hydroxy, halogen, nitro, cyano, heteroaryl, heteroaralkyl or thiol;
n is 0 or 1; and
Z' is a pharmaceutically acceptable anion, such as chloride, bromide, iodide, sulfate, methanesulfonate, p-toluenesulfonate, nitrate or phosphate.

Furthermore, the invention relates to a compound of formula II, which is the free base form of the compounds of formula I, provided R₄ is hydrogen 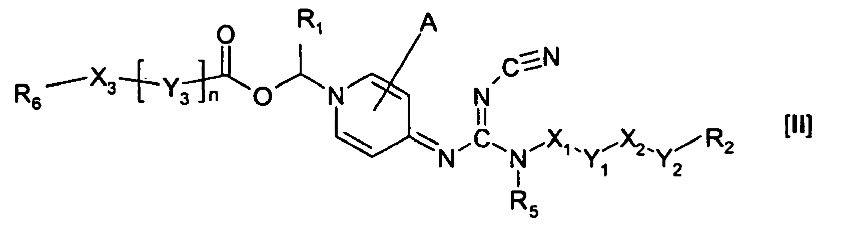 wherein A, R₁, R₂, R₅, R₆, X₁, X₂, X₃ Y₁, Y₂, Y₃ and n are as indicated above.

It is understood that the compounds of the present invention include any tautomeric forms, optical isomers or diastereoisomers thereof, either in pure form or as mixtures thereof. It is further understood that the invention includes pharmaceutically acceptable salts of compounds of formula I or II.

On administration of a compound of formula I or formula II to a patient, the ester or carbonate group R₆-X₃-(Y₃)ₙ-C(O)O-CHR₁- is hydrolysed enzymatically to liberate the active compound of formula III 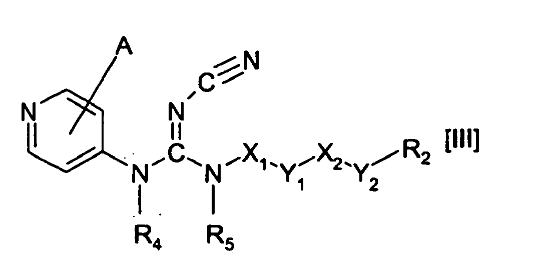 wherein A, R₂, R₄, R₅, X₁, X₂, Y₁, and Y₂ are as indicated above, together with the aldehyde R₁CHO.

### DETAILED DESCRIPTION OF THE INVENTION

### Definitions

In the present context, the term "prodrug" is intended to indicate a derivative of an active compound which does not, or does not necessarily, exhibit the physiological activity of the active compound, but which may be subjected to enzymatic cleavage such as hydrolysis *in vivo* so as to release the active compound on administration of the prodrug. In this particular instance, the prodrug comprises the active compound which in itself is highly lipophilic provided with a side chain with predominantly hydrophilic properties imparting improved solubility characteristics to the prodrug, thereby making it more suitable for parenteral administration in the form of a solution or for oral administration to obtain an improved bioavailability. More specifically, the hydrophilic side chain selected for the compounds of the present invention comprises an ester or carbonate group of formula R₆-X₃-(Y₃)ₙ-C(O)O-CHR₁- (wherein R₆, R₁, X₃, Y₃ and n are as indicated above).

The term "alkyl" is intended to indicate a univalent radical derived from straight, branched or cyclic alkane by removing a hydrogen atom from any carbon atom, preferably comprising from 1-8 carbon atoms. The term includes the subclasses primary, secondary and tertiary alkyl, such as methyl, ethyl, n-propyl, isopropyl, n-butyl, sec.-butyl, isobutyl, tert.-butyl, isopentyl, isohexyl, cyclohexyl, cyclopentyl and cyclopropyl.

The term "aryl" is intended to indicate radicals of carbocyclic aromatic rings, optionally fused bi-, tri- or tetra-cyclic rings wherein at least one ring is aromatic, e.g. phenyl, naphthyl, indanyl, indenyl, 1,4-dihydronaphtyl, flourenyl or tetralinyl.

The term "heteroaryl" is intended to indicate radicals of heterocyclic aromatic rings, in particular 5- or 6-membered rings with 1-3 heteroatoms selected from O, S and N, or optionally fused bicyclic rings, of which at least one is aromatic, with 1-4 heteroatoms, e.g. pyrrolyl, furanyl, thiophenyl, imidazolyl, oxazolyl, thiazolyl, pyrazolyl, pyridyl, pyrimidyl, purinyl, quinolinyl, chromenyl or carbazolyl.

The term "aralkyl" is intended to indicate an aromatic ring with an alkyl side chain as defined above, e.g. benzyl.

The term "halogen" is intended to indicate fluoro, chloro, bromo or iodo.

The term "aminosulfonyl" indicates a radical of the formula -S(O)₂NR^{a}₂, wherein each R^{a} independently represents either hydrogen or alkyl as defined above.

The term "alkylsulfonylamino" indicates a radical of the formula -NR^{a}₂-S(O)₂-R^{b}, wherein each R^{a} independently represents hydrogen or alkyl as defined above, and R^{b} represents alkyl as defined above.

The term "alkylcarbonyl" indicates a radical of the formula -C(O)R^{b}, wherein R^{b} is as just described.

The term "amino" indicates a radical of the formula -N(R^{a})₂, wherein each R^{a} independently represents hydrogen or alkyl as defined above.

The term "alkylcarbonylamino" indicates a radical of the formula -NR^{a}C(O)R^{b}, wherein R^{a} and R^{b} are as just described.

The term "alkoxy" indicates a radical of the formula OR^{b}, wherein R^{b} is as just described.

The term "alkoxycarbonyl" is intended to indicate a radical of the formula -C(O)-OR^{b}, wherein R^{b} is as indicated above.

The term "aminoacylamino" is intended to indicate a radical of the formula -NH-C(O)-R^{c}-NH₂, wherein R^{c} is a diradical known from any natural amino acid, H₂N-R^{c}-COOH, or its enantiomer.

The term "aminocarbonyl" is intended to indicate a radical of the formula -C(O)-NR^{a}₂, wherein each R^{a} independently represent hydrogen or alkyl as defined above.

The term "alkoxycarbonylamino" is intended to indicate a radical of the formula -NR^{a}-C(O)-OR^{b}, wherein R^{a} and R^{b} are as indicated above.

The term "hydrocarbon" is intended to indicate a compound comprising only hydrogen and carbon atoms, it may contain one or more double or triple carbon-carbon bonds, and it may comprise cyclic moieties in combination with branched or linear moieties. Said hydrocarbon preferably comprises 1-18, e.g. 1-12 carbon atoms. The term may be qualified as "non-aromatic heterocyclic", which is intended to indicate saturated or partly saturated cyclic compounds with 1-3 heteroatoms selected from O, S or N or optionally fused bicyclic rings with 1-4 heteroatoms, such as pyrrolidinyl, 3-pyrrolinyl, tetrahydrofuranyl, tetrahydrothiophenyl, piperidinyl, piperazinyl.

The term "a heterocyclic ring or condensed ring system with 3-10 ring atoms, wherein at least 1 ring atom constitutes an aliphatic amine" is intended to include radicals, such as pyrrolidinyl, piperidyl, hexahydro-1H-azapinyl, imidazolidinyl, piperazinyl, decahydro-isoquinolinyl, octahydro-isoindolyl, 1,2,3,4-tetrahydro-isoquinolinyl, 2,3-dihydro-1H-isoindolyl and morpholinyl

The term "pharmaceutically acceptable salt" is intended to indicate salts prepared by reacting a compound of formula I or II comprising a basic group with a suitable inorganic or organic acid, e.g. hydrochloric, hydrobromic, hydroiodic, sulfuric, nitric, acetic, phosphoric, lactic, maleic, phthalic, citric, propionic, benzoic, glutaric, gluconic , methanesulfonic, salicylic, succinic, tartaric, toluenesulfonic, sulfamic or fumaric acid.

### Preferred embodiments of the compound of formula I or II

In a preferred embodiment of the invention, X₂ and Y₁ are both bonds, while X₁ is a straight, branched or cyclic, saturated or unsaturated hydrocarbon diradical with 4 to 20 carbon atoms; Y₂ is O, S, C(O) or a bond;
R₂ is aryl or heteroaryl, both of which are optionally substituted by one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxy, cyano or nitro; tetrahydropyranyloxy, di-(C₁₋₄ alkoxy)phosphinoyloxy or C₁₋₄ alkoxycarbonylamino ;
X₃ is a straight hydrocarbon diradical comprising from 1 to 10 carbon atoms. R₆ is -NH₂ or piperidyl, attached at the 2, 3 or 4 position to X₃, and in particular at the 3 or 4 position;
R₁ is hydrogen, straight or branched C₁₋₄alkyl, aralkyl or aryl;
A, R₄ and R₅ are all hydrogen;
n is 0 or 1;
and Z⁻ is a pharmaceutically acceptable anion, such as chloride, bromide, iodide, sulfate, methanesulfonate, p-toluenesulfonate or nitrate.

In a preferred embodiment of the compounds of formula I or II, R₂ is aryl and in particular phenyl, optionally substituted by one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxy, cyano or nitro. A particular preferred substituent is halogen, such as chloro.

In a preferred embodiment of the compounds of formula I or II, Y₁ is a bond and Y₂ is O.

In a further preferred embodiment of the compounds of formula I or II, X₁ is a C₄₋₁₂ hydrocarbon diradical and X₂ is a bond.

Examples of specific compounds of formula I are
1-[2-(4-Piperidyl)-ethoxy-carbonyloxymethyl]-4-[N'-cyano-N'' -(6-(4-chloro-phenoxy)-1-hexyl)-N-guanidino]-pyridinium chloride, hydrochloride;
1-[3-Piperidyl-methoxy-carbonyloxymethyl]-4-[N'-cyano-N'' -(6-(4-chlorophenoxy)-1-hexyl)-N-guanidino]-pyridinium chloride, hydrochloride;
1-[4-Piperidyl-methoxy-carbonyloxymethyl]-4-[N'-cyano-N' -(6-(4-chlorophenoxy)-1-hexyl)-N-guanidino]-pyridinium chloride, hydrochloride;
1-[8-Amino-l-octyloxy-carbonyloxymethyl]-4-[N'-cyano-N''-(6-(4-chlorophenoxy)-1-hexyl)-N-guanidino]-pyridinium chloride, hydrochloride;
1-[4-Piperidyl-carbonyloxymethyl]-4-[N'-cyano-N''-(6-(4-chloro-phenoxy)-1-hexyl)-N-guanidino]-pyridinium chloride, hydrochloride;

As described above, an advantage of the prodrug forms of cyanoguanidines of the present invention is an increased solubility compared to the solubility of the cyanoguanidines themselves. The cause for said increase lies with at least two factors, i.e. the positive charge at the pyridine nitrogen, and the hydrophilic character of the prodrog moiety, i.e. 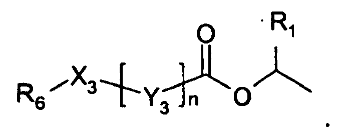 Pyridines in general have pK_{B} values around 9. This indicates that if pH is raised from acid pH values, e.g. 3 to physiological pH then the compounds of the present invention will be transformed from compounds of formula I to the corresponding free base, i.e. to compounds of formula II. At physiological pH, the positive charge at the pyridine nitrogen has thus largely disappeared, and this will lower the solubility of the compounds. It is believed to be a particular advantage of the compounds of the present invention that the prodrug moiety at R₆ bears a unit charge, or at least a fraction of a unit charge, at physiological pH. As defined, R₆ comprises an aliphatic amine moiety, and it is well-known that aliphatic amines have pK_{B} values in the 3-5 range [Frenna, J.Chem.Soc. Perkin Trans. II, 1865, 1985], which implies that the amine moiety is mainly protonated at physiological pH. The protonation gives rise to a charge which increases solubility.

Moreover, the following compounds is found to be particular useful in the preparation of compounds of formula I and II
Chloromethyl 2-(1-(tert-butoxycarbonyl)-4-piperidyl)-ethyl carbonate;
Chloromethyl 1-(tert-butoxycarbonyl)-3-piperidyl-methyl carbonate;
Chloromethyl 1-(tert-butoxycarbonyl)-4-piperidyl-methyl carbonate;
Iodomethyl 2-(1-(tert-butoxycarbonyl)-4-piperidyl)-ethyl carbonate;
Iodomethyl 1-(tert-butoxycarbonyl)-3-piperidyl-methyl carbonate;
Iodomethyl 1-(tert-butoxycarbonyl)-4-piperidyl-methyl carbonate;
Chloromethyl 8-(tert-butoxycarbonylamino)-1-octyl carbonate;
Iodomethyl 8-(tert-butoxycarbonylamino)-1-octyl carbonate;
Chloromethyl N- tert-butoxycarbonyl-4-piperidylcarboxylate;
Iodomethyl N-tert-butoxycarbonyl-4-piperidylcarboxylate;
1-[2-[1-(tert-Butoxycarbonyl)-4-piperidyl]-ethoxy-carbonyloxymethyl]-4-[N'-cyano-N''-(6-(4-chlorophenoxy)-1-hexyl)-N-guanidino]-pyridinium iodide;
1-[1-(tert-Butoxycarbonyl)-3-piperidyl-methoxy-carbonyloxymethyl]-4-[N' - cyano-N'' -(6-(4-chlorophenoxy)-1-hexyl)-N-guanidino]-pyridinium iodide;
1-[1-(tert-Butoxycarbonyl)-4-piperidyl-methoxy-carbonyloxymethyl]-4-[N' - cyano-N'' -(6-(4-chlorophenoxy)-1-hexyl)-N-guanidino]-pyridinium iodide;
1-[8-(N-tert-Butoxycarbonylamino)-1-octyloxy-carbonyloxymethyl]-4-[N' - cyano-N'' -(6-(4-chlorophenoxy)-1-hexyl)-N-guanidino]-pyridinium iodide; and
1-[1-(tert-Butoxycarbonyl)-4-piperidyl-carbonyloxymethyl]-4-[N' -cyano-N'' -(6-(4-chlorophenoxy)-1-hexyl)-N-guanidino]-pyridinium iodide;

### General methods of preparation

Compounds of formula I may be prepared by reacting a compound of formula III 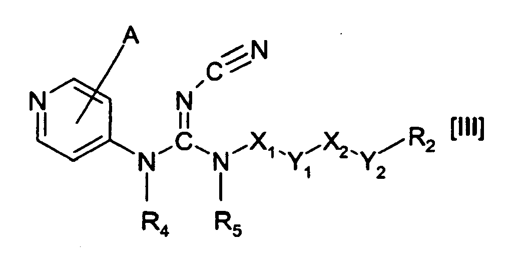 wherein A, R₂, R₄, R₅, X₁, X₂, Y₁ and Y₂ are as indicated in formula I, with a compound of formula IV 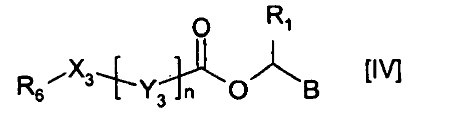 wherein R₁, R₆, X₃, Y₃, and n are as indicated above, and B is a leaving group, such as Cl, Br or I. In addition R₆ and X₃ may optionally contain protecting-groups.

The reaction of a compound of formula III with a compound of formula IV may be performed in a solvent-free environment or In an inert solvent such as acetonitrile at a temperature between room temperature and 150°C to afford a compound of formula I optionally after removal of protecting groups.

The compounds of formula IV are known from the literature or may be prepared by methods well known to persons skilled in the art.

When n is 1, compounds of formula IV may be prepared by reacting a compound of formula V 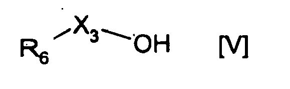 wherein R₆ and X₃ are as indicated in formula IV, with a compound of formula VI 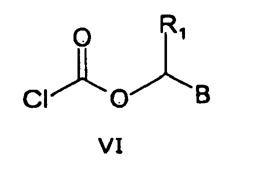 wherein R₁ and B are as indicated above.

The reaction between a compound of formula V and a compound of formula VI may be performed at a temperature between room temperature and - 70°C in an inert organic solvent, such as dichloromethane, in the presence of a suitable base such as pyridine.

When n is zero, compounds of formula IV in which B is chlorine may be prepared by reacting a compound of formula VII 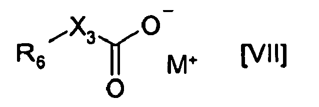 wherein R₆ and X₃ are as indicated in formula IV and M⁺ is a suitable metal kation, e. g. an alkalimetal kation, or a tertiary ammonium ion, with a compound of formula VIII

X-CH(R₁)-Cl VIII

wherein R₁ is as indicated above and X is lodo, bromo or chlorosulfonyloxy.

The reaction between VII and VIII may be performed in a suitable solvent such as dimethylformamide at a suitable temperature, e.g. at room temperature, when X is iodo or bromo. When X is chlorosulfonyloxy the reaction may be performed under phase transfer conditions as described in Synthetic Communications 14, 857-864 (1984).

Compounds of formula IV in which B is chloro may be transformed into the corresponding compounds in which B is lodo by reaction with sodium iodide in acetone or acetonitrile.

The compounds of formulae V, VI, VII, VIII are either known from the literature or may be prepared by methods well known to persons skilled in the art.

Compounds of formula III are known from the literature and may be prepared by any one of the methods disclosed in, for instance, EP 660 823, WO 98/54141, WO 98/54143, WO 98/54144, WO 98/54145, WO 00/61559 and WO 00/61561.

A compound of formula I, provided that R₄ is hydrogen may be converted into the corresponding free base of formula II by treating a solution of a compound of formula I in an appropriate inert solvent, e.g. dichloromethane, with a suitable base, e.g. aqueous sodium bicarbonate. The free base of formula II may be reconverted into a salt of formula I by treating a solution of a compound of formula II in an appropriate inert solvent, e.g. dichloromethane, with a suitable acid of formula ZH, wherein Z is as Indicated above.

### Pharmaceutical compositions

In another aspect, the invention relates to pharmaceutical formulations of a compound of formula I or II intended for the treatment of proliferative diseases. The present invention thus provides a pharmaceutical composition comprising a compound of formula I or II together with a pharmaceutically acceptable excipient or diluent. The formulations of the present invention, both for veterinary and for human medical use, comprise active ingredients in association with a pharmaceutically acceptable carrier(s) and optionally other therapeutic ingredient(s). The carrier(s) must be "acceptable" in the sense of being compatible with the other ingredients of the formulations and not deleterious to the recipient thereof.

Conveniently, the active ingredient comprises from 0.1-100% by weight of the formulation. Conveniently, a dosage unit of a formulation contain between 0.07 mg and 1 g of a compound of formula I or II.

By the term "dosage unit" is meant a unitary, i.e. a single dose which is capable of being administered to a patient, and which may be readily handled and packed, remaining as a physically and chemically stable unit dose comprising either the active material as such or a mixture of it with solid or liquid pharmaceutical diluents or carriers.

The formulations include e.g. those in a form suitable for oral (including sustained or timed release), rectal, parenteral (including subcutaneous, intraperitoneal, intramuscular, intraarticular and intravenous), transdermal, ophthalmic, topical, nasal or buccal administration.

The formulations may conveniently be presented in dosage unit form and may be prepared by any of the methods well known in the art of pharmacy, e.g as disclosed in Remington, The Science and Practice of Pharmacy, 20th ed., 2000. All methods include the step of bringing the active ingredient into association with the carrier, which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units, such as capsules, sachets, tablets or lozenges, each containing a predetermined amount of the active ingredient; in the form of a powder or granules; in the form of a solution or a suspension in an aqueous liquid or non-aqueous liquid, such as ethanol or glycerol; or in the form of an oil-in-water emulsion or a water-in-oil emulsion. Such oils may be edible oils, such as e.g. cottonseed oil, sesame oil, coconut oil or peanut oil. Suitable dispersing or suspending agents for aqueous suspensions include synthetic or natural gums such as tragacanth, alginate, acacia, dextran, sodium carboxymethylcellulose, gelatin, methylcellulose, hydroxypropylmethylcellulose, hydroxypropylcellulose, carbomers and polyvinylpyrrolidone. The active ingredients may also be administered in the form of a bolus, electuary or paste.

A tablet may be made by compressing or moulding the active ingredient optionally with one or more accessory ingredients. Compressed tablets may be prepared by compressing, in a suitable machine, the active Ingredient(s) in a free-flowing form such as a powder or granules, optionally mixed by a binder, such as e.g. lactose, glucose, starch, gelatine, acacia gum, tragacanth gum, sodium alginate, carboxymethylcellulose, methylcellulose, hydroxypropylmethylcellulose, polyethylene glycol, waxes or the like; a lubricant such as e.g. sodium oleate, sodium stearate, magnesium stearate, sodium benzoate, sodium acetate, sodium chloride or the like; a disintegrating agent such as e.g. starch, methylcellulose, agar, bentonite, croscarmellose sodium, sodium starch glycollate, crospovidone or the like or a dispersing agent, such as polysorbate 80. Moulded tablets may be made by moulding, in a suitable machine, a mixture of the powdered active ingredient and suitable carrier moistened with an inert liquid diluent.

Formulations for rectal administration may be may in the form of suppositories in which the compound of the present invention is admixed with low melting water soluble or insoluble solids such as cocoa butter, hydrogenated vegetable oils, polyethylene glycol or fatty acids esters of polyethylene glycols, while elixirs may be prepared using myristyl palmitate.

Formulations suitable for parenteral administration conveniently comprise a sterile oily or aqueous preparation of the active ingredients, which is preferably isotonic with the blood of the recipient, e.g. isotonic saline, isotonic glucose solution or buffer solution. The formulation may be conveniently sterilised by for instance filtration through a bacteria retaining filter, addition of sterilising agent to the formulation, irradiation of the formulation or heating of the formulation. Liposomal formulations as disclosed in e.g. Encyclopedia of Pharmaceutical Technology, vol.9, 1994, are also suitable for parenteral administration.

Alternatively, the compound of formula I may be presented as a sterile, solid preparation, e.g. a freeze-dried powder, which is readily dissolved in a sterile solvent immediately prior to use.

Transdermal formulations may be in the form of a plaster or a patch.

Formulations suitable ophthalmic administration may be in the form of a sterile aqueous preparation of the active ingredients, which may be in microcrystalline form, for example, in the form of an aqueous microcrystalline suspension. Liposomal formulations or biodegradable polymer systems e.g. as disclosed in Encyclopedia of Pharmaceutical Technology, vol.2, 1989, may also be used to present the active ingredient for ophthalmic administration.

Formulations suitable for topical or ophthalmic administration include liquid or semi-liquid preparations such as liniments, lotions, gels, applicants, oil-in-water or water-in-oil emulsions such as creams, ointments or pastes; or solutions or suspensions such as drops.

Formulations suitable for nasal or buccal administration include powder, self-propelling and spray formulations, such as aerosols and atomisers.

In addition to the aforementioned ingredients, the formulations of a compound of formula I or II may Include one or more additional ingredients such as diluents, buffers, flavouring agents, colourant, surface active agents, thickeners, preservatives, e.g. methyl hydroxybenzoate (including anti-oxidants), emulsifying agents and the like.

In the systemic treatment using the present invention daily doses of from 0.001-500 mg per kilogram body weight, preferably from 0.002-100 mg/kg of mammal body weight, for example 0.003-20 mg/kg or 0.003 to 5 mg/kg of a compound of formula I or II is administered, typically corresponding to a daily dose for an adult human of from 0.01 to 37000 mg. However, the present invention also provides compounds and compositions intended for administration with longer intervals, e.g. every week, every three weeks or every month. In the topical treatment of dermatological disorders, ointments, creams or lotions containing from 0.1-750 mg/g, and preferably - from 0.1-500 mg/g, for example 0.1-200 mg/g of a compound of formula I or II is administered. For topical use in ophthalmology ointments, drops or gels containing from 0.1-750 mg/g, and preferably from 0.1-500 mg/g, for example 0.1-200 mg/g of a compound of formula I or II is administered. The oral compositions are formulated, preferably as tablets, capsules, or drops, containing from 0.07-1000 mg, preferably from 0.1-500 mg, of a compound of formula I or II per dosage unit.

In a preferred embodiment, the invention provides pharmaceutical compositions comprising a compound of formula I or II in combination with one or more other pharmacologically active compounds used in the treatment of proliferative diseases. Examples of compounds used in the treatment of proliferative diseases which may be used together with compounds of the present invention include S-triazine derivatives such as altretamine; enzymes such as asparaginase; antibiotic agents such as bleomycin, dactinomycin, daunorubicin, doxorubicin, idarubicin, mitomycin, epirubicin and plicamycin; alkylating agents such as busulfan, carboplatin, carmustine, chlorambucil, cisplatin, cyclophosphamide, dacarbazine, ifosfamide, lomustine, mechlorethamine, melphalan, procarbazine and thiotepa; antimetabolites such as cladribine, cytarabine, floxuridine, fludarabine, fluorouracil, hydroxyurea, mercaptopurine, methotrexate, gemcitabin, pentostatin and thioguanine; antimitotic agents such as etoposide, paclitaxel, teniposide, vinblastine, vinorelbin and vincristine; hormonal agents, e.g. aromatase inhibitors such as aminoglutethimide, corticosteroids, such as dexamethasone and prednisone, and luteinizing hormone releasing hormone (LH-RH); antiestrogens such as tamoxifen, formestan and letrozol; antiandrogens such as flutamide; biological response modifiers, e.g. lymphokines such as aldesleukin and other interleukines; interferon such as interferon-a; growth factors such as erythropoietin, filgrastim and sagramostim; differentiating agents such as vitamin D derivatives, e.g. seocalcitol, and all-trans retinoic acid; immunoregulators such as levamisole; and monoclonal antibodies, tumour necrosis factor α and angiogenesis inhibitors. Finally, ionising radiation, although not readily defined as a compound, is heavily depended on in the treatment of neoplastic diseases, and may be combined with the compounds of the present Invention. Due to the severe side effects often experienced by patients receiving anti-neoplastic treatment it is often desirable also to administer therapeutics which are not in themselves anti-neoplastic, but rather help relieving the side effects of anti-neoplastic therapy. Such compounds include amifostin, leucovorin and mesna.

In particular, anti-neoplastic compounds, such as paclitaxel, fluorouracil, etoposide, cyclophospamide, cisplatin, carboplatin, vincristine, gemcitabine, vinorelbine, chlorambucil, doxorubicin and melphalan appear beneficial in the combination compositions of the present invention.

It is envisaged that the combination composition of the present invention may be provided as mixtures of the compounds or as individual compounds intended for simultaneous or sequential administration. It lies within the capabilities of a skilled physician or veterinarian to decide time intervals in a sequential administration regime. The invention thus also provides a pharmaceutical composition comprising, in separate containers and for sequential or simultaneous administration, a compound of formula I or II and one or more other anti-neoplastic compound(s), together with pharmaceutically acceptable excipients or diluents.

In a further aspect, the invention concerns a method of treating or ameliorating proliferative diseases or conditions, the method comprising administering, to a patient in need thereof, a pharmaceutical composition comprising a compound of formula I or II, which compound is hydrolysed enzymatically upon administration to provide a compound of formula III, in an amount sufficient to effect treatment or amelioration of said proliferative disease or condition, optionally together with another anti-neoplastic compound and/or ionising radiation. The invention thus provides a compound of formula I or II for use as a medicament.

In particular, proliferative diseases or conditions to be treated by the present method include a variety of cancers and neoplastic diseases or conditions including leukaemia, acute myeloid leukaemia, chronic myeloid leukaemia, chronic lymphatic leukaemia, myelodysplasia, multiple myeloma, Hodgkin's disease or non-Hodgkin's lymphoma, small or non-small cell lung carcinoma, gastric, intestinal or colorectal cancer, prostrate, ovarian or breast cancer, brain, head or neck cancer, cancer in the urinary tract, kidney or bladder cancer, malignant melanoma, liver cancer, uterine or pancreatic cancer.

Cyanoguanidines are also believed to be useful in the treatment of inflammatory diseases. In one aspect, the invention thus provides a method of treating inflammatory diseases, the method comprising administering to a patient an effective amount of a compound of the present invention, either alone or in combination with other therapeutically active compounds.

The invention also relates to the use of compounds of formula I or II, optionally together with one or more other anti-neoplastic compounds, as indicated above, for the preparation of a medicament for the treatment or amelioration of proliferative diseases or conditions, e.g. cancers as mentioned above. The invention also provides a compound of formula I or II, optionally together with one or more other antineoplastic compounds, for use in the treatment or amelioration of proliferative diseases or conditions.

As indicated above, it is preferred to administer the compounds of the invention parenterally, such as in a liquid, preferably aqueous, solution intended for intravenous injection or infusion. A suitable dosage of the compound of the invention will depend, inter alia, on the age and condition of the patient, the severity of the disease to be treated and other factors well known to the practising physician. The compound may be administered either orally or parenterally according to different dosing schedules, e.g. daily or with weekly intervals. In general a single dose will be in the range from 0.1 to 400 mg/kg bodyweight. Parenterally, the compound may be administered as a bolus (i.e. the entire dose is administered at once) or in divided doses two or more times a day or preferably as an intravenous infusion.

The invention is described in further detail in the following examples which are not in any way intended to limit the scope of the invention as claimed.

### EXAMPLES

For ¹H nuclear magnetic resonance (NMR) spectra (300 MHz) and ¹³C NMR (75.6 MHz) chemical shift values are quoted relative to Internal tetramethylsilane (δ=0.00) or chloroform (δ=7.25) or deuteriochloroform (δ =76.81 for ¹³C NMR) standards. The value of a multiplet, either defined (singlet (s), doublet (d), triplet (t), quartet (q)) or not (broad (br)), at the approximate midpoint is given unless a range is quoted. The organic solvents used were anhydrous.

### Preparation 1

### Chloromethyl 2-(1-(tert-butoxycerbonyl)-4-piperidyl)-ethyl carbonate

Pyridine (3.22 ml) was added to an ice-cold solution of 2-(1-(tert-butoxycarbonyl)-4-piperidyl)-ethanol (7.6 g) in dichloromethane (33 ml) followed by chloromethyl chloroformate (3.23 ml) at such a rate that the temperature was kept below 10°C. After stirring overnight at room temperature the-reaction mixture was washed twice with 0.5 M HCl followed by water and aqueous sodium bicarbonate. The organic phase was dried over magnesium sulfate, filtered and evaporated *in vacuo* to yield the title compound as a colourless oil.
¹H NMR (CDCl₃) δ = 5.73 (s, 2H), 4.28 (t, 2H), 4.09 (d, 2H), 2.69 (t, 2H), 1.75-1.50 (m, 5H), 1.45 (s, 9H), 1.15 (m, 2H)

### Preparation 2

### Chloromethyl 1-(tert-butoxycarbonyl)-3-piperidyl-methyl carbonate

Prepared as described in Preparation 1 but substituting 1-(tert-butoxycarbonyl)-3-piperidyl-methanol for 2-(1-(tert-butoxycarbonyl)-4-piperidyl)-ethanol. Light yellow oil.
¹H NMR (CDCl₃) δ = 5.73 (s, 2H), 4.11 (m, 2H), 4.0-3.75 (m, 2H), 3.00-2.60 (m, 2H), 2.00-1.60 (m, 3H), 1.45 (s, 9H), 1.29 (m, 2H)

### Preparation 3

### Chloromethyl 1-(tert-butoxycarbonyl)-4-piperidyl-methyl carbonate

Prepared as described in Preparation 1 but substituting 1-(tert-butoxycarbonyl)-4-piperidyl-methanol for 2-(1-(tert-butoxycarbonyl)-4-piperidyl)-ethanol. Light yellow oil.
¹H NMR (CDCl₃) δ = 5.73 (s, 2H), 4.13 (d, 2H), 4.09 (d, 2H), 2.71 (d, 2H), 1.88 (m, 1H), 1.71 (d, 2H), 1.45 (s, 9H), 1.21 (m, 2H)

### Preparation 4

### Iodomethyl 2-(1-(tert-butoxycarbonyl)-4-piperidyl)-ethyl carbonate

Chloromethyl 2-(1-(tert-butoxycarbonyl)-4-piperidyl)-ethyl carbonate (4.9 g) was added to a solution of sodium iodide (9 g) in acetone (20 ml). After stirring at 40 °C for 2.5 hours the reaction mixture was cooled to room temperature, filtered and evaporated in vacuo. The residue was taken up in dichloromethane, washed with aqueous sodium bicarbonate and sodium thiosulfate, dried over magnesium sulfate, filtered and evaporated *in vacuo.* Purification on silica gel with hexane/ethyl acetate (2: 1) as eluent gave the title compound as a light yellow oil.
¹H NMR (CDCl₃) δ = 5.95 (s, 2H), 4.28 (t, 2H), 4.09 (d, 2H), 2.69 (t, 2H), 1.75-1.50 (m, 5H), 1.45 (s, 9H), 1.13 (m, 2H)

### Preparation 5

### Iodomethyl 1-(tert-butoxycarbonyl)-3-piperidyl-methyl carbonate

Prepared as described in Preparation 4 but substituting chloromethyl 1-(tert-butoxycarbonyl)-3-piperidyl-methyl carbonate for chloromethyl 2-(1-(tert-butoxycarbonyl)-4-piperidyl)-ethyl carbonate. Light yellow oil.
¹H NMR (CDCl₃) δ = 5.95 (s, 2H), 4.11 (m, 2H), 4.0-3.75 (m, 2H), 3.00-2.60 (m, 2H), 2.00-1.60 (m, 3H), 1.45 (s, 9H), 1.29 (m, 2H)

### Preparation 6

### Iodomethyl 1-(tert-butoxycarbonyl)-4-piperidyl-methyl carbonate

Prepared as described in Preparation 4 but substituting chloromethyl 1-(tert-butoxycarbonyl)-4-piperidyl-methyl carbonate for chloromethyl 2-(1-(tert-butoxycarbonyl)-4-piperidyl)-ethyl carbonate. Light yellow oil.
¹H NMR (CDCl₃) δ = 5.95 (s, 2H), 4.13 (m, 2H), 4.08 (d, 2H), 2.70 (t, 2H), 1.87 (m, 1H), 1.70 (d, 2H), 1.46 (s, 9H), 1.20 (m, 2H)

### Preparation 7

### Chloromethyl 8-(tert-butoxycarbonylamino)-1-octyl carbonate

A solution of pyridine (1.57 ml) and 8-(tert-butoxycarbonylamino)-1-octanol (4 g) in dichloromethane (40 ml) was cooled in dry-ice . Precipitation occurred during cooling and chloromethyl chloroformate (1.6 ml) was added to the stirred suspension at such a rate that the temperature was kept below -50 °C. After stirring for 2 hours below -50 °C the cooling bath was removed and the temperature was allowed to rise to room temperature. The mixture was washed twice with 0.5 M HCl followed by water, aqueous sodium bicarbonate and saturated sodium chloride. The organic phase was dried over magnesium sulfate, filtered and evaporated *in vacuo* to yield the title compound as a colourless oil.
¹H NMR (CDCl₃) δ = 5.73 (s, 2H), 4.52 (br, 1H), 4.22 (t, 2H), 3.10 (q, 2H), 1.69 (m, 2H), 1.44 (s, 9H), 1.55-1.20 (m, 10H)

### Preparation 8

### Iodomethyl 8-(tert-butoxycarbonylamino)-1-octyl carbonate

This compound was prepared as described in Preparation 4 but substituting chloromethyl 8-(tert-butoxycarbonylamino)-1-octyl carbonate for chloromethyl 2-(1-(tert-butoxycarbonyl)-4-piperidyl)-ethyl carbonate. Light yellow oil.
¹H NMR (CDCl₃) δ = 5.95 (s, 2H), 4.51 (br, 1H), 4.21 (t, 2H), 3.10 (q, 2H), 1.68 (m, 2H), 1.44 (s, 9H), 1.55-1.20 (m, 10H)

### Preparation 9

### Chloromethyl- N- tert-butoxycarbonyl-4-Diperidylcarboxylate

To a solution of N- tert-butoxycarbonyl-4-piperidyl-carboxylic acid (6.4 g) in dichloromethane (30 ml) was added water (30 ml), sodium bicarbonate (8.91 g) and tetrabutylammonium hydrogensulfate (0.95 g). The mixture was stirred at room temperature while chloromethyl chlorosulfate (3.19 ml) was added slowly. After stirring for a further 30 minutes the organic phase was separated and evaporated *in vacuo.* The crude product was distributed between diethyl ether and water. The organic phase was separated, dried and evaporated to yield the title compound as an oil.
**¹³C** NMR (CDCl₃) δ = 172.6, 154.6, 79.7, 68.7, 42.8, 40.8, 28.4, 27.5

### Preparation 10

### Iodomethyl N-tert-butoxycarbonyl-4-piperidylcarboxylate

Prepared as described in Preparation 4 but substituting chloromethyl N-tert-butoxycarbonyl-4-piperidylcarboxylate for chloromethyl 2-(1-(tert-butoxycarbonyl)-4-piperidyl)-ethyl carbonate. Light yellow oil.
¹³C NMR (CDCl₃) δ = 172.6, 154.6, 79.7, 42.8, 41.0, 30.5, 28.4, 27.4

### Preparation 11

### 1-[2-[1-(tert-Butoxycarbonyl)-4-piperidyl]-ethoxy-carbonyloxymethyl]-4-[N'-cyano-N''-(6-4-chlorophenoxy)-1-hexyl)-N-quanidino]-pyridinium iodide.

Iodomethyl 2-[1-(tert-butoxycarbonyl)-4-piperidyl]-ethyl carbonate (5 g) was added to a hot solution of N-(6-(4-chlorophenoxy)-1-hexyl)-N'-cyano-N' -(4-pyridyl)-guanidine (2.8 g) in dry acetonitrile (110 ml) followed by reflux for 20 minutes. After cooling to room temperature and concentration *in vacuo,* the title compound crystallised and was isolated by filtration. Recrystallisation from acetonitrile gave the title compound as light yellow crystals.
¹H NMR (CDCl₃) δ = 11.24 (br, 1H), 8.58 (d, 2H), 8.24 (br, 2H), 7.81 (br, 1H), 7.20 (d, 2H), 6.82 (d, 2H), 6.19 (s, 2H), 4.26 (t, 2H), 4.08 (d, 2H), 3.94 (t, 2H), 3.77 (q, 2H), 2.67 (t, 2H), 1.78 (m, 4H), 1.64 (m, 4H), 1.52 (m, 5H), 1.44 (s, 9H), 1.14 (m, 2H)

### Preparation 12

### 1-[1-(tert-Butoxycarbonyl)-3-piperidyl-methoxy-carbonyloxymethyl]-4-N'-cyano-N''-(6-(4-chlorophenoxy)-1-hexyl)-N-guanidino]-pyridinium iodide.

Iodomethyl 1-(tert-butoxycarbonyl)-3-piperidyl-methyl carbonate (5.4 g) was added to a hot solution of N-(6-(4-chlorophenoxy)-1-hexyl)-N'-cyano-N''-(4-pyridyl)-guanidine (2.8 g) in dry acetonitrile (110 ml) followed by reflux for 20 minutes. After cooling to room temperature and concentration *in vacuo,* ethyl acetate was added and the title compound crystallised and was isolated by filtration. Recrystallisation from ethyl acetate gave the title compound as light yellow crystals.
¹H NMR (CDCl₃) δ = 11.28 (br, 1H), 8.57 (d, 2H), 8.27 (br, 2H), 7.85 (br, 1H), 7.20 (d, 2H), 6.82 (d, 2H), 6.19 (s, 2H), 4.10 (d, 2H), 3.94 (t, 2H), 3.87 (m, 2H), 3.79 (m, 2H), 2.93 (m, 1H), 2.71 (m, 1H), 2.00-1.48 (m, 11H), 1.44 (s, 9H), 1.26 (m, 2H)

### Preparation 13

### 1-[1-(tert-Butoxycarbonyl)-4-piperidyl-methoxy-carbonyloxymethyl]-4-[N'-cyano-N''-(6-(4-chlorophenoxy)-1-hexyl)-N-guanidino]-pyridinium iodide.

Iodomethyl 1-(tert-butoxycarbonyl)-4-piperidyl-methyl carbonate (9 g) was added to a hot solution of N-(6-(4-chlorophenoxy)-1-hexyl)-N'-cyano-N'' - (4-pyridyl)-guanidine (4 g) in dry acetonitrile (160 ml) followed by reflux for 20 minutes. After cooling to room temperature and concentration *in vacuo,* ethyl acetate was added and the title compound crystallised and was isolated by filtration as a light yellow powder.
¹H NMR (CDCl₃) δ = 11.25 (br, 1H), 8.57 (d, 2H), 8.25 (br, 2H), 7.96 (br, 1H), 7.19 (d, 2H), 6.83 (d, 2H), 6.19 (s, 2H), 4.12 (br, 2H), 4.06 (d, 2H), 3.93 (t, 2H), 3.77 (t, 2H), 2.69 (t, 2H), 1.93-1.48 (m, 11H), 1.45 (s, 9H), 1.18 (m,2H)

### Preparation 14

### 1-[8-(N-tert-Butoxycarbonylamino)-1-octyloxy-carbonyloxymethyl]-4-[N'-cyano-N''-(6-(4-chlorophenoxy)-1-hexyl)-N-quanidino]-pyridinium iodide.

Iodomethyl 8-(tert-butoxycarbonylamino)-1-octyl carbonate (5.47 g) was added to a hot solution of N-(6-(4-chlorophenoxy)-1-hexyl)-N'-cyano-N''-(4-pyridyl)-guanidine (3.16 g) in dry acetonitrile (140 ml) followed by reflux for 20 minutes. After cooling to room temperature the title compound crystallised and after further cooling in ice the crystalline product was isolated by filtration.
¹³C NMR (CDCl₃) δ = 157.7, 156.0, 154.9, 153.8, 143.8, 129.2, 125.2, 115.8, 114.4, 80.4, 79.1, 70.5, 68.0, 43.0, 40.5, 30.0, 29.2, 29.0, 28.9, 28.4, 28.2, 26.6, 26.3, 25.4, 25.4

### Preparation 15

### 1-[1-(tert-Butoxycarbonyl)-4-piperidyl-carbonyloxymethyl]-4-[N-cyano-N''-6-(4-chlorophenoxy)-1-hexyl)-N-guanidino]-pyridinium iodide.

Iodomethyl N-tert-butoxycarbonyl-4-piperidylcarboxylate (6.52 g) was added to a hot solution of N-(6-(4-chlorophenoxy)-1-hexyl)-N'-cyano-N''-(4-pyridyl)-guanidine (4.38 g) in dry acetonitrile (170 ml) followed by reflux for 20 minutes. After cooling to room temperature and concentration *in vacuo* the title compound crystallised and after further cooling in ice the crystalline product was isolated by filtration.
¹³C NMR (CDCl₃) δ = 173.5, 157.7, 154.6, 154.5, 153.9, 143.9, 129.3, 125.2, 115.9, 114.5, 80.0, 77.7, 68.0, 43.0, 40.6, 29.2, 28.9, 28.4, 27.6, 26.3, 25.5

### Example 1

### 1-[2-(4-Piperidyl)-ethoxy-carbonyloxymethyl]-4-[N'-cyano-N''-(6-(4-chloro-phenoxy)-1-hexyl)-N-guanidino]-pyridinium chloride, hydrochloride.

A solution of 1-[2-[1-(tert-butoxycarbonyl)-4-piperidyl]-ethoxy-carbonyloxy-methyl]-4-[N'-cyano-N''-(6-(4-chlorophenoxy)-1-hexyl)-N-guanldlno]-pyridinium iodide (1.9 g) in dichloromethane (60 ml) was shaken with an excess of aqueous sodium bicarbonate and sodium thiosulfate. The organic phase was dried over magnesium sulfate and filtered. After concentration *in vacuo* to about 15 ml the clear filtrate was cooled in ice with stirring and treated with an excess of hydrogen chloride in ether. The ice bath was removed and after stirring for 4 hours, the solvent was removed *in vacuo.* The residue crystallised upon addition of ethanol and after recrystallisation from methanol/ether the title compound was obtained in the form of nice colourless crystals.
¹H NMR (DMSO) δ = 12.10 (br, 1H), 9.18 (br, 2H), 8.94 (br, 1H), 8.75 (d, 2H), 7.58 (br, 2H), 7.31 (d, 2H), 6.95 (d, 2H), 6.22 (s, 2H), 4.20 (t, 2H), 3.96 (t, 2H), 3.41 (m, 2H), 3.19 (m, 2H), 2.78 (q, 2H), 1.86-1.25 (m, 15H)

### Example 2

### 1-[3-Piperidyl-methoxy-carbonyloxymethyl]-4-[N'-cyano-N''-(6-(4-chloro-phenoxy)-1-hexyl)-N-guanidino]-pyridinium chloride, hydrochloride.

A solution of 1-[1-(tert-butoxycarbonyl)-3-piperidyl-methoxy-carbonyloxymethyl]-4-[N'-cyano-N''-(6-(4-chlorophenoxy)-1-hexyl)-N-guanidino]-pyridinium iodide (1.7 g) in dichloromethane (60 ml) was shaken with an excess of aqueous sodium bicarbonate and sodium thiosulfate. The organic phase was dried over magnesium sulfate and filtered. After concentration *in vacuo* to about 15 ml the clear filtrate was cooled in ice with stirring and treated with an excess of hydrogen chloride in ether. The ice bath was removed and after stirring for 4 hours, the solvent was removed *in vacuo.* The residue crystallised upon addition of ethanol and after filtration and recrystallisation from methanol/ether the title compound was obtained as colourless crystals.
¹H NMR (DMSO) δ = 12.10 (br, 1H), 9.27 (br, 3H), 8.76 (d, 2H), 7.61 (br, 2H), 7.31 (d, 2H), 6.95 (d, 2H), 6.24 (s, 2H), 4.09 (m, 2H), 3.96 (t, 2H), 3.41 (br, 2H), 3.18 (d, 2H), 2.65 (m, 2H), 2.18 (br, 1H), 1.85-1.13 (m, 12H)

### Example 3

### 1-[4-Piperidyl-methoxy-carbonyloxymethyl]-4-[N'-cyano-N''-(6-(4-chloro-phenoxy)-1-hexyl)-N-guanidino]-pyridinium chloride, hydrochloride.

A solution of 1-[1-(tert-butoxycarbonyl)-4-piperidyl-methoxy-carbonyloxymethyl]-4-[N'-cyano-N''-(6-(4-chlorophenoxy)-1-hexyl)-N-guanidino]-pyridinium iodide (1 g) in dichloromethane (30 ml) was shaken with an excess of aqueous sodium bicarbonate and sodium thiosulfate. The organic phase was dried over magnesium sulfate and filtered. After concentration *in vacuo* to about 15 ml the clear filtrate was cooled in ice with stirring and treated with an excess of hydrogen chloride in ether. The ice bath was removed and after stirring for 4 hours, the solvent was removed *in vacuo* to yield the title compound as a colourless foam.
¹H NMR (DMSO) δ = 12.05 (br, 1H), 9.19 (br, 2H), 8.93 (br, 1H), 8.78 (d, 2H), 7.65 (br, 2H), 7.31 (d, 2H), 6.95 (d, 2H), 6.24 (s, 2H), 4.50 (d, 2H), 3.96 (t, 2H), 3.42 (q, 2H), 3.22 (d, 2H), 2.82 (m, 2H), 1.8-1.25 (m, 13H)

### Example 4

### 1-[8-Amino-2-octyloxy-carbonyloxymethyl]-4-[N'-cyano-N''-(6-(4-chlorophenoxy)-1-hexyl)-N-guanidino]-pyridinium chloride, hydrochloride

Prepared as described in Example 1 but substituting 1-[8-(N-tert-butoxycarbonylamino)-1-octyloxy-carbonyloxymethyl]-4-[N'-cyano-N''-(6-(4-chlorophenoxy)-1-hexyl)-N-guanidino]-pyridinium iodide for 1-[2-[1-(tert-butoxycarbonyl)-4-piperidyl]-ethoxy-carbonyloxy-methyl]-4-[N' - cyano-N''-(6-(4-chlorophenoxy)-1-hexyl)-N-guanidino]-pyridinium iodide. Light yellow crystals.
¹³C NMR (DMSO) δ = 157.4, 154.9, 153.0, 144.9, 129.1, 123.9, 116.1, 115.0, 112.7, 80.1, 68.9, 67.6, 42.1, 28.3, 28.2, 27.7, 26.7, 25.7, 25.6, 25.0, 24.8

### Example 5

### 1-[4-Piperidyl-carbonyloxymethyl]-4-[N'-cyano-N''-(6-(4-chloro-phenoxy)-1-hexyl)-N-quanidino]-pyridinium chloride, hydrochloride.

Prepared as described in Example 2 but substituting 1-[1-(tert-butoxycarbonyl)-4-piperidyl-carbonyloxymethyl]-4-[N' -cyano-N'' -(6-(4-chlorophenoxy)-1-hexyl)-N-guanidino]-pyridinium iodide for 1-[2-[1-(tert-butoxycarbonyl)-4-piperidyl]-ethoxy-carbonyloxy-methyl]-4-[N'-cyano-N'-(6-(4-chlorophenoxy)-1-hexyl)-N-guanidino]-pyridinium lodide. Crystalline powder.
¹³C NMR (DMSO) δ = 172.2, 157.4, 154.8, 144.8, 129.1, 123.9, 116.1, 115.0, 78.1, 67.6, 41.7, 37.2, 28.3, 25.7, 25.0, 23.9

## Claims

1. A compound according to formula I 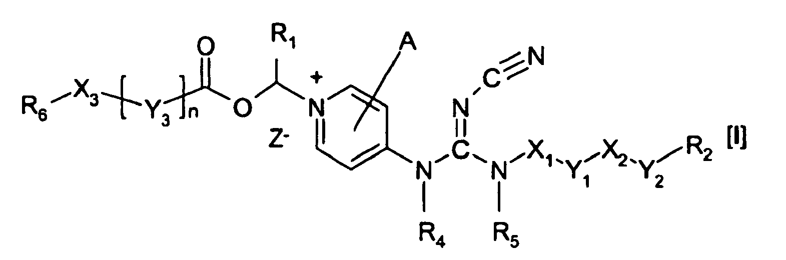 wherein X₁ is a straight, branched and/or cyclic hydrocarbon diradical, optionally substituted with one or more hydroxy, halogen, nitro, amino or cyano;
X₂ is a bond; a straight, branched and/or cyclic hydrocarbon diradical, optionally substituted with one or more hydroxy, halogen, nitro, amino, cyano, aminosulfonyl, alkylsulfonylamino, alkylcarbonyl, formyl, aminocarbonyl or alkylcarbonylamino; a heteroarylene or non-aromatic heterocyclic hydrocarbon diradical, all of which are optionally substituted with one or more straight, branched and/or cyclic non-aromatic hydrocarbon radical, hydroxyl, halogen, amino, nitro, cyano, aminosulfonyl, alkylsulfonylamino, alkylcarbonyl, formyl, aminocarbonyl or alkylcarbonylamino;
X₃ is a straight, branched and/or cyclic hydrocarbon diradical, optionally substituted with one or more substituents selected from the group consisting of hydroxy, halogen, nitro, amino, cyano, aminosulfonyl, alkylsulfonylamino, alkylcarbonyl, formyl, aminocarbonyl or alkylcarbonylamino; with the proviso that when n is 0, X₃ may also be a bond;
Y₁ is a bond, O, S, S(O), S(O)₂, C(O), NH-C(O) or C(O)-NH;
Y₂ is a bond, an ether diradical (R'-O-R"), an amine diradical (R'-N-R"), O, S, S(O), S(O)₂, C(O), NH-C(O), C(O)-NH, SO₂-N(R') or N(R')-SO₂ wherein R' and R" are independently straight or branched hydrocarbon diradicals containing up to 4 carbon atoms;
Y₃ is O;
R₁ is hydrogen or straight, branched and/or cyclic alkyl, optionally substituted with phenyl; or an aromatic hydrocarbon radical;
R₂ is hydrogen, or aryl or heteroaryl, both of which are optionally substituted with one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxyl, cyano or nitro; tetrahydropyranyloxy, di-(C₁₋₄ alkoxy)phosphinoyloxy or C₁₋₄ alkoxycarbonylamino;
R₄ is hydrogen;
R₅ is hydrogen, or a straight, branched and/or cyclic hydrocarbon radical, optionally substituted with halogen, hydroxyl, halogen, amino, nitro or cyano;
R₆ is piperidyl, attached at the 3 or 4 position to X₃;
A is hydrogen, an optionally substituted, straight, branched and/or cyclic hydrocarbon radical, hydroxy, halogen, nitro, cyano, heteroaryl, heteroaralkyl or thiol;
n is 0 or 1; and
Z⁻ is a pharmaceutically acceptable anion, such as chloride, bromide, iodide, sulfate, methanesulfonate, p-toluenesulfonate, nitrate or phosphate.

2. A compound of the general formula II 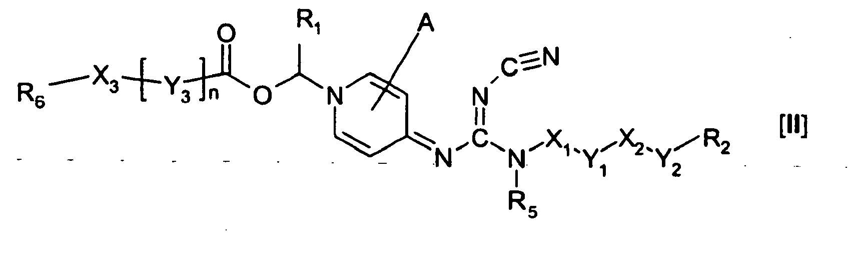 wherein A, X₁, X₂, X₃, Y₁, Y₂, Y₃, R₁, R₂, R₅, R₆ and n are as indicated in claim 1.

3. A compound according to claim 1 or 2 wherein X₂ and Y₁ are both bonds;
X₁ is a straight, branched or cyclic, saturated or unsaturated hydrocarbon diradical with 4 to 20 carbon atoms;
Y₂ is O, S, C(O) or a bond;
R₂ is aryl or heteroaryl, both of which are optionally substituted by one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxy, cyano or nitro; tetrahydropyranyloxy, di-(C₁₋₄ alkoxy)phosphinoyloxy or C₁₋₄ alkoxycarbonylami no;
X₃ is a straight hydrocarbon diradical comprising from 1 to 10 carbon atoms;
R₁ is hydrogen, straight or branched C₁₋₄alkyl, aralkyl or aryl;
A, R₄ and R₅ are all hydrogen;
n is 0 or 1;
and Z⁻ is chloride, bromide, iodide, sulfate, methanesulfonate, p-toluenesulfonate or nitrate.

4. A compound according to claim 1-3, wherein R₂ is aryl optionally substituted by one or more substituent selected from the group consisting of halogen, trifluoromethyl, hydroxy, C₁₋₄alkoxy, nitro, cyano, C₁₋₄hydroxyalkyl or C₁₋₄alkyl, optionally substituted with halogen, hydroxy, cyano or nitro.

5. A compound according to any of claims 1-3, wherein R₂ is phenyl or phenyl substituted by one or more substituent selected from the group consisting of halogen, trifluoromethyl, hydroxy, C₁₋₄alkoxy, nitro, cyano, C₁₋₄ hydroxyalkyl or C₁₋₄alkyl, optionally substituted with halogen, hydroxyl, cyano or nitro.

6. A compound according to claim 5, wherein said substituent is chloro.

7. A compound according to any claims 1-6, wherein Y₁ is a bond, and Y₂ is O.

8. A compound according to any claims 1-7, wherein X₁ is a C₄₋₁₂ hydrocarbon diradical, and X₂ is a bond.

9. A compound according to claim 1 which is selected from the group consisting of
1-[2-(4-Piperidyl)-ethoxy-carbonyloxymethyl]-4-[N'-cyano-N''-(6-(4-chloro-phenoxy)-1-hexyl)-N-guanidino]-pyridinium chloride, hydrochloride; 1-[3-Piperidyl-methoxy-carbonyloxymethyl]-4-[N' -cyano-N" -(6-(4-chloro-phenoxy)-1-hexyl)-N-guanidino]-pyridinium chloride, hydrochloride; 1-[4-Piperidyl-methoxy-carbonyloxymethyl]-4-[N'-cyano-N" -(6-(4-chloro-phenoxy)-1-hexyl)-N-guanidino]-pyridinium chloride, hydrochloride; 1-[4-Piperidyl-carbonyloxymethyl]-4-[N'-cyano-N'' -(6-(4-chloro-phenoxy)-1-hexyl)-N-guanidino]-pyridinium chloride, hydrochloride.

10. 1-[8-Amino-1-octyloxy-carbonyloxymethyl]-4-[N' -cyano-N" -(6-(4-chlorophenoxy)-1-hexyl)-N-guanidino]-pyridinium chloride, hydrochloride.

11. A pharmaceutical composition comprising a compound of formula I or II according to any one of claims 1-10 together with a pharmaceutically acceptable excipient or diluent.

12. A composition according to claim 11, wherein the compound is dissolved in an appropriate, pharmaceutically acceptable solvent, e.g. selected from the group consisting of water, isotonic saline, isotonic glucose solution, or a buffer solution.

13. A composition according to claim 12 for parenteral administration, intravenous injection or infusion.

14. A composition according to any one of claims 11-13 further comprising one or more other anti-neoplastic compounds.

15. A composition according to claim 14, wherein said other antineoplastic compound(s) is selected from the group consisting of S-triazin derivatives, antibiotic agents, alkylating agents, anti-metabolites, anti-mitotic agents, hormonal agents, differentiating agents, biological response modifiers and angiogenesis inhibitors.

16. A composition according to claim 15, wherein the compound of formula I or II 1-[2-(4-Piperidyl)-ethoxy-carbonyloxymethyl]-4-[N' -cyano-N''-(6-(4-chloro-phenoxy)-1-hexyl)-N-guanidino]-pyridinium chloride, hydrochloride, and wherein the other anti-neoplastic agent(s) is selected from the group consisting of paclitaxel, fluorouacil, etoposide, cyclophosphamide, cisplatin, carboplatin, vincristine, gemcitabine, vinorelbine, chlorambucil, doxorubicin, melphalan and seocalcitol.

17. A pharmaceutical composition comprising, in separate containers and intended for sequential or simultaneous administration, a compound of formula I or II according to any one of claims 1-10 and one or more other anti-neoplastic compound(s), together with pharmaceutically acceptable exipients or diluents.

18. A compound according to any one of claims 1-10 for use as a medicament.

19. Use of a compound of formula I or II according to any one of claims 1-10, optionally together with one or more other anti-neoplastic compounds, for the preparation of a medicament for the treatment or amelioration of proliferative diseases or conditions.

20. The use according to claim 19, wherein the proliferative disease is cancer.

21. The use according to claim 19, wherein said proliferative disease is selected from the group consisting of leukaemia, acute myeloid leukaemia, chronic myeloid leukaemia, chronic lymphatic leukaemia, myelodysplasia, multiple myeloma, Hodgkin's disease or non-Hodgkin's lymphoma, small or non-small cell lung carcinoma, gastric, intestinal or colorectal cancer, prostate, ovarian or breast cancer, head, brain or neck cancer, cancer in the urinary tract, kidney or bladder cancer, malignant melanoma, liver cancer, uterine or pancreatic cancer.

22. The use according to any one of claims 19-21, wherein said other anti-neoplastic compound(s) is selected from the group consisting of S-triazin derivatives, antibiotic agents, alkylating agents, anti- metabolites, anti-mitotic agents, hormonal agents, differentiating agents, biological response modifiers and angiogenesis inhibitors.

23. The use according to any one of claims 19-22, wherein the compound of formula I or II is 1-[2-(4-Piperidyl)-ethoxy-carbonyloxymethyl]-4-[N'-cyano-N' -(6-(4-chloro-phenoxy)-1-hexyl)-N-guanidino]-pyridinium chloride, hydrochloride, and wherein said other anti-neoplastic compound is selected from the group consisting of paclitaxel, fluorouacil, etoposide, cyclophosphamide, cisplatin, carboplatin, vincristine, gemcitabine, vinorelbine, chlorambucil, doxorubicin, melphalan and seocalcitol.

24. A compound selected from the group consisting of Chloromethyl 2-(1-(tert-butoxycarbonyl)-4-piperidyl)-ethyl carbonate; Chloromethyl 1-(tert-butoxycarbonyl)-3-piperidyl-methyl carbonate; Chloromethyl 1-(tert-butoxycarbonyl)-4-piperidyl-methyl carbonate; Iodomethyl 2-(1-(tert-butoxycarbonyl)-4-piperidyl)-ethyl carbonate; Iodomethyl 1-(tert-butoxycarbonyl)-3-piperidyl-methyl carbonate; Iodomethyl 1-(tert-butoxycarbonyl)-4-piperidyl-methyl carbonate; Chloromethyl 8-(tert-butoxycarbonylamino)-1-octyl carbonate; Iodomethyl 8-(tert-butoxycarbonylamino)-1-octyl carbonate; Chloromethyl N-tert-butoxycarbonyl-4-piperidylcarboxylate; Iodomethyl N-tert-butoxycarbonyl-4-piperidylcarboxylate; 1-[2-[1-(tert-Butoxycarbonyl)-4-piperidyl]-ethoxy-carbonyloxymethyl]-4-[N'-cyano-N" -(6-(4-chlorophenoxy)-1-hexyl)-N-guanidino]-pyridinium iodide;
1-[1-(tert-Butoxycarbonyl)-3-piperidyl-methoxy-carbonyloxymethyl]-4-[N' - cyano-N'' -(6-(4-chlorophenoxy)-1-hexyl)-N-guanidino]-pyridinium iodide; 1-[1-(tert-Butoxycarbonyl)-4-piperidyl-methoxy-carbonyloxymethyl]-4-[N' - cyano-N' ' -(6-(4-chlorophenoxy)-1-hexyl)-N-guanidino]-pyridinium iodide; 1-[8-(N-tert-Butoxycarbonylamino)-1-octyloxy-carbonyloxymethyl]-4-[N' - cyano-N' ' -(6-(4-chlorophenoxy)-1-hexyl)-N-guanidino]-pyridinium iodide; and
1-[1-(tert-Butoxycarbonyl)-4-piperidyl-carbonyloxymethyl]-4-[N'-cyano-N''-(6-(4-chlorophenoxy)-1-hexyl)-N-guanidino]-pyridinium iodide.

25. A compound according to any one of claims 1-10, optionally together with one or more other anti-neoplastic compounds, for use in the treatment or amelioration of proliferative diseases or conditions.

## Patentansprüche

1. Verbindung gemäß der Formel I 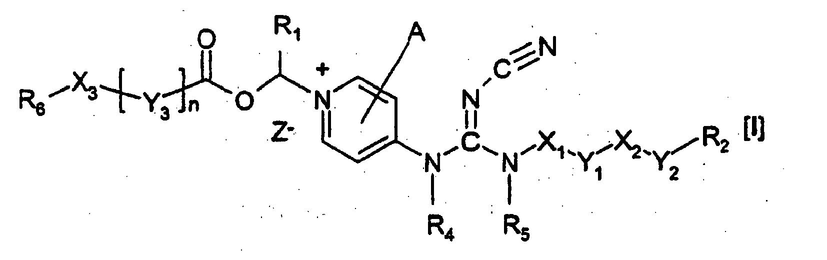 worin X₁ eine geradkettige, verzweigte und/oder cyclische zweiwertige Kohlenwasserstoffgruppe ist, die gegebenenfalls mit einem oder mehreren von Hydroxy, Halogen, Nitro, Amino oder Cyano substituiert ist;
X₂ eine Bindung; eine geradkettige, verzweigte und/oder cyclische zweiwertige Kohlenwasserstoffgruppe, die gegebenenfalls mit einem oder mehreren von Hydroxy, Halogen, Nitro, Amino, Cyano, Aminosulfonyl, Alkylsulfonylamino, Alkylcarbonyl, Formyl, Aminocarbonyl oder Alkylcarbonylamino substituiert ist; eine zweiwertige Heteroarylengruppe oder nichtaromatische heterocyclische Kohlenwasserstoffgruppe, die alle gegebenenfalls mit einer oder mehreren von einer geradkettigen, verzweigten und/oder cyclischen nicht-aromatischen Kohlenwasserstoffgruppe, Hydroxyl, Halogen, Amino, Nitro, Cyano, Aminosulfonyl, Alkylsulfonylamino, Alkylcarbonyl, Formyl, Aminocarbonyl oder Alkylcarbonylamino substituiert sind, ist;
X₃ eine geradkettige, verzweigte und/oder cyclische zweiwertige Kohlenwasserstoffgruppe ist, die gegebenenfalls mit einem oder mehreren Substituenten substituiert ist, der bzw. die aus der Gruppe, bestehend aus Hydroxy, Halogen, Nitro, Amino, Cyano, Aminosulfonyl, Alkylsulfonylamino, Alkylcarbonyl, Formyl, Aminocarbonyl oder Alkylcarbonylamino, ausgewählt ist bzw. sind, mit der Maßgabe, dass dann, wenn n 0 ist, X₃ auch eine Bindung sein kann;
Y₁ eine Bindung, O, S, S(O), S(O)₂, C(O), NH-C(O) oder C(O)-NH ist;
Y₂ eine Bindung, eine zweiwertige Ethergruppe (R'-O-R"), eine zweiwertige Amingruppe (R'-N-R"), O, S, S(O), S(O)₂, C(O), NH-C(O), C(O)-NH, SO₂-N(R') oder N(R')-SO₂ ist, worin R' und R" unabhängig geradkettige oder verzweigte zweiwertige Kohlenwasserstoffgruppen sind, die bis zu 4 Kohlenstoffatome enthalten;
Y₃ O ist;
R₁ Wasserstoff oder ein geradkettiges, verzweigtes und/oder cyclisches Alkyl, das gegebenenfalls mit Phenyl substituiert ist, oder eine aromatische Kohlenwasserstoffgruppe ist;
R₂ Wasserstoff oder Aryl oder Heteroaryl, die beide gegebenenfalls mit einem oder mehreren Substituenten substituiert sind, der bzw. die aus der Gruppe, bestehend aus Halogen, Trifluormethyl, Hydroxy, C₁₋₄-Alkoxy, Nitro, Cyano, C₁₋₄-Hydroxyalkyl oder C₁₋₄-Alkyl, gegebenenfalls substituiert mit Halogen, Hydroxyl, Cyano oder Nitro, ausgewählt ist bzw. sind; Tetrahydropyranyloxy, Di-(C₁₋₄-alkoxy)phosphinoyloxy oder C₁₋₄-Alkoxycarbonylamino ist;
R₄ Wasserstoff ist;
R₅ Wasserstoff oder eine geradkettige, verzweigte und/oder cyclische Kohlenwasserstoffgruppe ist, die gegebenenfalls mit Halogen, Hydroxyl, Halogen, Amino, Nitro oder Cyano substituiert ist;
R₆ Piperidyl ist, das an der 3- oder 4-Position an X₃ gebunden ist;
A Wasserstoff, eine gegebenenfalls substituierte geradkettige, verzweigte und/oder cyclische Kohlenwasserstoffgruppe, Hydroxy, Halogen, Nitro, Cyano, Heteroaryl, Heteroaralkyl oder Thiol ist;
n 0 oder 1 ist und
Z⁻ ein pharmazeutisch verträgliches Anion, wie z.B. Chlorid, Bromid, Iodid, Sulfat, Methansulfonat, p-Toluolsulfonat, Nitrat oder Phosphat ist.

2. Verbindung der allgemeinen Formel II 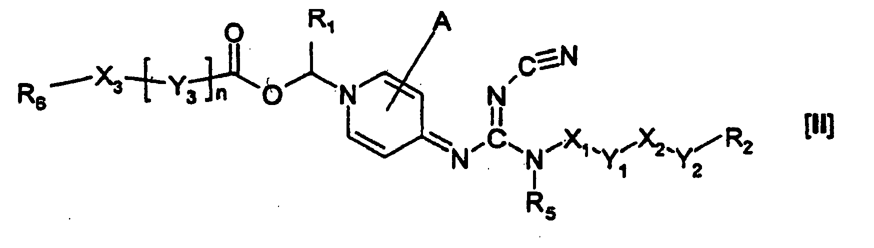 worin A, X₁, X₂, X₃, Y₁, Y₂, Y₃, R₁, R₂, R₅, R₆ und n die im Anspruch 1 angegebene Bedeutung haben.

3. Verbindung nach Anspruch 1 oder 2, bei der X₂ und Y₁ beide Bindungen sind;
X₁ eine geradkettige, verzweigte oder cyclische, gesättigte oder ungesättigte, zweiwertige Kohlenwasserstoffgruppe mit 4 bis 20 Kohlenstoffatomen ist;
Y₂ O, S, C(O) oder eine Bindung ist; R₂ Aryl oder Heteroaryl, die beide gegebenenfalls mit einem oder mehreren Substituenten substituiert sind, der bzw. die aus der Gruppe, bestehend aus Halogen, Trifluormethyl, Hydroxy, C₁₋₄₋Alkoxy, Nitro, Cyano, C₁₋₄-Hydroxyalkyl oder C₁₋₄-Alkyl, gegebenenfalls substituiert mit Halogen, Hydroxy, Cyano oder Nitro, ausgewählt ist bzw. sind; Tetrahydropyranyloxy, Di-(C₁₋₄-alkoxy)phosphinoyloxy oder C₁₋₄-Alkoxycarbonylamino ist;
X₃ eine geradkettige zweiwertige Kohlenwasserstoffgruppe ist, die 1 bis 10 Kohlenstoffatome umfasst;
R₁ Wasserstoff, geradkettiges oder verzweigtes C₁₋₄-Alkyl, -Aralkyl oder-Aryl ist;
A, R₄ und R₅ alle Wasserstoff sind;
n 0 oder 1 ist; und
Z⁻ Chlorid, Bromid, Iodid, Sulfat, Methansulfonat, p-Toluolsulfonat oder Nitrat ist.

4. Verbindung nach einem der Ansprüche 1 bis 3, bei der R₂ Aryl ist, das gegebenenfalls mit einem oder mehreren Substituenten substituiert ist, der bzw. die aus der Gruppe, bestehend aus Halogen, Trifluormethyl, Hydroxy, C₁₋₄-Alkoxy, Nitro, Cyano, C₁₋₄-Hydroxyalkyl oder C₁₋₄-Alkyl, gegebenenfalls substituiert mit Halogen, Hydroxy, Cyano oder Nitro, ausgewählt ist bzw. sind.

5. Verbindung nach einem der Ansprüche 1 bis 3, bei der R₂ Phenyl oder Phenyl ist, das gegebenenfalls mit einem oder mehreren Substituenten substituiert ist, der bzw. die aus der Gruppe, bestehend aus Halogen, Trifluormethyl, Hydroxy, C₁₋₄-Alkoxy, Nitro, Cyano, C₁₋₄-Hydroxyalkyl oder C₁₋₄-Alkyl, gegebenenfalls substituiert mit Halogen, Hydroxyl, Cyano oder Nitro, ausgewählt ist bzw. sind.

6. Verbindung nach Anspruch 5, bei welcher der Substituent Chlor ist.

7. Verbindung nach einem der Ansprüche 1 bis 6, bei der Y₁ eine Bindung und Y₂ O ist.

8. Verbindung nach einem der Ansprüche 1 bis 7, bei der X₁ eine zweiwertige C₄₋₁₂-Kohlenwasserstoffgruppe und X₂ eine Bindung ist.

9. Verbindung nach Anspruch 1, die aus der Gruppe, bestehend aus 1-[2-(4-Piperidyl)ethoxycarbonyloxymethyl]-4-[N'-cya no-N "-(6-(4-chlorphenoxy)-1-hexyl)-N-guanidino]pyridiniumchlorid, -hydrochlorid; 1-[3-Piperidylmethoxycarbonyloxymethyl]-4-[N'-cyano-N"-(6-(4-chlorphenoxy)-1-hexyl)-N-guanidino]pyridiniumchlorid, -hydrochlorid; 1-[4-Piperidylmethoxycarbonyloxymethyl]-4-[N'-cyano-N "-(6-(4-chlorphenoxy)-1-hexyl)-N-guanidino]pyridiniumchlorid, -hydrochlorid; 1-[4-Piperidylcarbonyloxymethyl]-4-[N'-cyano-N"-(6-(4-chlorphenoxy)-1-hexyl)-N-guanidino]pyridiniumchlorid, -hydrochlorid, ausgewählt ist.

10. 1-[8-Amino-1-octyloxycarbonyloxymethyl]-4-[N'-cyano-N"-(6-(4-chlorphenoxy)-1-hexyl)-N-guanidino]pyridiniumchlorid, -hydrochlorid.

11. Pharmazeutische Zusammensetzung, die eine Verbindung der Formel I oder II gemäß einem der Ansprüche 1 bis 10 zusammen mit einem pharmazeutisch verträglichen Träger oder Verdünnungsmittel umfasst.

12. Zusammensetzung nach Anspruch 11, bei der die Verbindung in einem geeigneten, pharmazeutisch verträglichen Lösungsmittel, das z.B. aus der Gruppe, bestehend aus Wasser, isotonischer Kochsalzlösung, isotonischer Glukoselösung oder einer Pufferlösung, ausgewählt ist, gelöst ist.

13. Zusammensetzung nach Anspruch 12 zur parenteralen Verabreichung, intravenösen Injektion oder Infusion.

14. Zusammensetzung nach einem der Ansprüche 11 bis 13, die ferner eine oder mehrere andere antineoplastische Verbindung(en) umfasst.

15. Zusammensetzung nach Anspruch 14, bei der die andere(n) antineoplastische(n) Verbindung(en) aus der Gruppe, bestehend aus S-Triazinderivaten, antibiotischen Mitteln, Alkylierungsmitteln, Antimetaboliten, antimitotischen Mitteln, Hormonmitteln, Differenzierungsmitteln, Mitteln zur Modifizierung einer biologischen Reaktion und Angiogeneseinhibitoren, ausgewählt ist bzw. sind.

16. Zusammensetzung nach Anspruch 15, bei der die Verbindung der Formel I oder II 1-[2-(4-Piperidyl)ethoxycarbonyloxymethyl]-4-[N'-cyano-N"-(6-(4-chlorphenoxy)-1-hexyl)-N-guanidino]pyridiniumchlorid, -hydrochlorid ist und bei der das bzw. die andere(n) antineoplastische(n) Mittel aus der Gruppe, bestehend aus Paclitaxel, Fluoruracil, Etoposid, Cyclophosphamid, Cisplatin, Carboplatin, Vincristin, Gemcitabin, Vinorelbin, Chlorambucil, Doxorubicin, Melphalan und Seocalcitol, ausgewählt ist bzw. sind.

17. Pharmazeutische Zusammensetzung, umfassend in separaten Behältern und vorgesehen für eine aufeinander folgende oder gleichzeitige Verabreichung, eine Verbindung der Formel I oder II gemäß einem der Ansprüche 1 bis 10 und eine oder mehrere andere antineoplastische Verbindung(en) zusammen mit pharmazeutisch verträglichen Trägern oder Verdünnungsmitteln.

18. Verbindung nach einem der Ansprüche 1 bis 10 zur Verwendung als Medikament.

19. Verwendung einer Verbindung der Formel I oder II gemäß einem der Ansprüche 1 bis 10, gegebenenfalls zusammen mit einer oder mehreren anderen antineoplastischen Verbindung(en), zur Herstellung eines Medikaments zur Behandlung oder Linderung proliferativer Erkrankungen oder Zustände.

20. Verwendung nach Anspruch 19, bei der die proliferative Erkrankung Krebs ist.

21. Verwendung nach Anspruch 19, bei der die proliferative Erkrankung aus der Gruppe, bestehend aus Leukämie, akuter myeloischer Leukämie, chronischer myeloischer Leukämie, chronischer lymphatischer Leukämie, Myelodysplasie, multiplem Myelom, Hodgkin-Krankheit oder non-Hodgkin-Lymphom, kleinzelligem oder nicht-kleinzelligem Lungenkarzinom, Magen-, Darm- oder Kolorektalkrebs, Prostata-, Eierstock- oder Brustkrebs, Kopf-, Gehirn- oder Nackentumor, Krebs im Harntrakt, Nieren- oder Blasenkrebs, malignem Melanom, Leberkrebs, Gebärmutter- oder Bauchspeicheldrüsenkrebs, ausgewählt ist.

22. Verwendung nach einem der Ansprüche 19 bis 21, bei der die andere(n) antineoplastische(n) Verbindung(en) aus der Gruppe, bestehend aus S-Triazinderivaten, antibiotischen Mitteln, Alkylierungsmitteln, Antimetaboliten, antimitotischen Mitteln, Hormonmitteln, Differenzierungsmitteln, Mitteln zur Modifizierung einer biologischen Reaktion und Angiogeneseinhibitoren, ausgewählt ist bzw. sind.

23. Verwendung nach einem der Ansprüche 19 bis 22, bei der die Verbindung der Formel I oder II 1-[2-(4-Piperidyl)ethoxycarbonyloxymethyl]-4-[N'-cyano-N"-(6-(4-chlorphenoxy)-1-hexyl)-N-guanidino]pyridiniumchlorid, -hydrochlorid ist und bei der die andere antineoplastische Verbindung aus der Gruppe, bestehend aus Paclitaxel, Fluoruracil, Etoposid, Cyclophosphamid, Cisplatin, Carboplatin, Vincristin, Gemcitabin, Vinorelbin, Chlorambucil, Doxorubicin, Melphalan und Seocalcitol, ausgewählt ist.

24. Verbindung, die aus der Gruppe, bestehend aus Chlormethyl-2-(1-(tert-butoxycarbonyl)-4-piperidyl)ethylcarbonat; Chlormethyl-1-(tert-butoxycarbonyl)-3-piperidylmethylcarbonat; Chlormethyl-1-(tert-butoxycarbonyl)-4-piperidylmethylcarbonat; lodmethyl-2-(1-(tert-butoxycarbonyl)-4-piperidyl)ethylcarbonat; lodmethyl-1-(tert-butoxycarbonyl)-3-piperidylmethylcarbonat; lodmethyl-1-(tert-butoxycarbonyl)-4-piperidylmethylcarbonat; Chlormethyl-8-(tert-butoxycarbonylamino)-1-octylcarbonat; lodmethyl-8-(tert-butoxycarbonylamino)-1-octylcarbonat; Chlormethyl-N-tert-butoxycarbonyl-4-piperidylcarboxylat; lodmethyl-N-tert-butoxycarbonyl-4-piperidylcarboxylat; 1-[2-[1-(tert-Butoxycarbonyl)-4-piperidyl]ethoxycarbonyloxymethyl)-4-[N'-cyano-N"-(6-(4-chlorphenoxy)-1-hexyl)-N-guanidino)pyridiniumiodid; 1-[1-(tert-Butoxycarbonyl)-3-piperidylmethoxycarbonyloxymethyl)-4-[N'-cyano-N"-(6-(4-chlorphenoxy)-1-hexyl)-N-guanidino]pyridiniumiodid; 1-[1-(tert-Butoxycarbonyl)-4-piperidylmethoxycarbonyloxymethyl]-4-[N'-cyano-N"-(6-(4-chlorphenoxy)-1-hexyl)-N-guanidino]-pyridiniumiodid; 1-[8-(N-tert-Butoxycarbonylamino)-1-octyloxycarbonyloxymethyl]-4-[N'-cyano-N"-(6-(4-chlorphenoxy)-1-hexyl)-N-guanidino]pyridiniumiodid und 1-[1-(tert-Butoxycarbonyl)-4-piperidylcarbonyloxymethyl]-4-[N'-cyano-N"-(6-(4-chlorphenoxy)-1-hexyl)-N-guanidino]pyridiniumiodid, ausgewählt ist.

25. Verbindung nach einem der Ansprüche 1 bis 10, gegebenenfalls zusammen mit einer oder mehreren anderen antineoplastischen Verbindung(en), zur Verwendung bei der Behandlung oder Linderung proliferativer Erkrankungen oder Zustände.

## Revendications

1. Composé selon la formule I 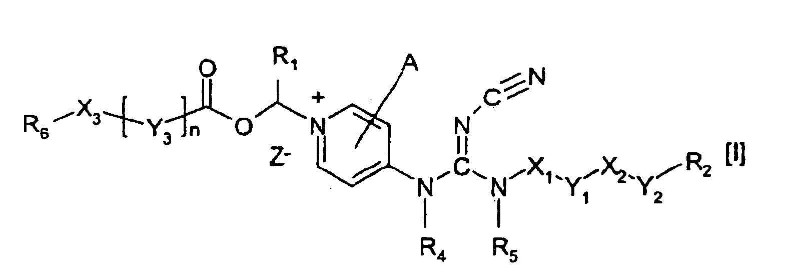 dans laquelle X₁ est un radical divalent hydrocarboné linéaire, ramifié et/ou cyclique, facultativement substitué par un ou plusieurs hydroxy, halogènes, nitro, amino ou cyano ;
X₂ est une liaison ; un radical divalent hydrocarboné linéaire, ramifié et/ou cyclique, facultativement substitué par un ou plusieurs hydroxy, halogènes, nitro, amino, cyano, aminosulfonyles, alkylsulfonylamino, alkylcarbonyles, formyles, aminocarbonyles ou alkylcarbonylamino ; un hétéroarylène ou un radical divalent hydrocarboné hétérocyclique non aromatique, tous étant facultativement substitués par un ou plusieurs radicaux hydrocarbonés linéaires, ramifiés et/ou cycliques non aromatiques, hydroxyles, halogènes, amino, nitro, cyano, aminosulfonyles, alkylsulfonylamino, alkylcarbonyles, formyles, aminocarbonyles ou alkylcarbonylamino ;
X₃ est un radical divalent hydrocarboné linéaire, ramifié et/ou cyclique, facultativement substitué par un ou plusieurs substituants choisis chacun entre un hydroxy, un halogène, un nitro, un amino, un cyano, un aminosulfonyle, un alkylsulfonylamino, un alkylcarbonyle, un formyle, un aminocarbonyle ou un alkylcarbonylamino ; à condition que lorsque n vaut 0, X₃ puisse également être une liaison ;
Y₁ est une liaison, O, S, S(O), S(O)₂, C(O), NH-C(O) ou C(O)-NH ;
Y₂ est une liaison, un radical éther divalent (R'-O-R"), un radical amine divalent (R'-N-R"), O, S, S(O), S(O)₂, C(O), NH-C(O), C(O)-NH, SO₂-N(R') ou N(R')-SO₂ dans lesquels R' et R" sont indépendamment des radicaux divalents hydrocarbonés linéaires ou ramifiés contenant jusqu'à 4 atomes de carbone ;
Y₃ est O ;
R₁ est un hydrogène ou un alkyle linéaire, ramifié et/ou cyclique, facultativement substitué par un phényle ; ou un radical hydrocarboné aromatique ;
R₂ est un hydrogène ou un aryle ou hétéroaryle, tous deux étant facultativement substitués par un ou plusieurs substituants choisis chacun entre un halogène, un trifluorométhyle, un hydroxy, un alcoxy en C₁₋₄, un nitro, un cyano, un hydroxyalkyle en C₁₋₄ ou un alkyle en C₁₋₄, facultativement substitué par un halogène, un hydroxyle, un cyano ou un nitro ; un tétrahydropyranyloxy, un di(alcoxy en C₁₋₄)phosphinoyloxy ou un alcoxycarbonylamino en C₁₋₄ ;
R₄ est un hydrogène ;
R₅ est un hydrogène ou un radical hydrocarboné linéaire, ramifié et/ou cyclique, facultativement substitué par un halogène, un hydroxyle, un halogène, un amino, un nitro ou un cyano ;
R₆ est un pipéridyle, attaché au niveau de la position 3 ou 4 à X₃ ;
A est un hydrogène, un radical hydrocarboné linéaire, ramifié et/ou cyclique facultativement substitué, un hydroxy, un halogène, un nitro, un cyano, un hétéroaryle, un hétéroaralkyle ou un thiol ;
n vaut 0 ou 1 ; et
Z⁻ est un anion acceptable du point de vue pharmaceutique, tel qu'un chlorure, un bromure, un iodure, un sulfate, un méthanesulfonate, un p-toluènesulfonate, un nitrate ou un phosphate.

2. Composé de formule générale II 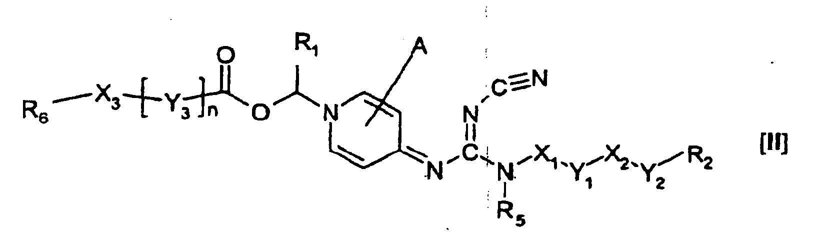 dans laquelle A, X₁, X₂, X₃, Y₁, Y₂, Y₃, R₁, R₂, R₅, R₆ et n sont tels qu'indiqués dans la revendication 1.

3. Composé selon la revendication 1 ou 2 dans lequel X₂ et Y₁ sont tous deux des liaisons ;
X₁ est un radical divalent hydrocarboné linéaire, ramifié ou cyclique saturé ou insaturé ayant 4 à 20 atomes de carbonée
Y₂ est O, S, C(O) ou une liaison ;
R₂ est un aryle ou hétéroaryle, tous deux étant facultativement substitués par un ou plusieurs substituants choisis chacun entre un halogène, un trifluorométhyle, un hydroxy, un alcoxy en C₁₋₄, un nitro, un cyano, un hydroxyalkyle en C₁₋₄ ou un alkyle en C₁₋₄, facultativement substitué par un halogène, un hydroxy, un cyano ou un nitro ; un tétrahydropyranyloxy, un di(alcoxy en C₁₋₄)phosphinoyloxy ou un alcoxycarbonylamino en C₁₋₄ ;
X₃ est un radical divalent hydrocarboné linéaire comprenant de 1 à 10 atomes de carbone ;
R₁ est un hydrogène, un alkyle en C₁₋₄ linéaire ou ramifié, un aralkyle ou un aryle ;
A, R₄ et R₅ sont tous des hydrogènes ;
n vaut 0 ou 1 ;
et Z ⁻ est un chlorure, un bromure, un iodure, un sulfate, un méthanesulfonate, un *p*-toluènesulfonate ou un nitrate.

4. Composé selon la revendication 1-3, dans lequel R₂ est un aryle facultativement substitué par un ou plusieurs substituants choisis chacun entre un halogène, un trifluorométhyle, un hydroxy, un alcoxy en C₁₋₄, un nitro, un cyano, un hydroxyalkyle en C₁₋₄ ou un alkyle en C₁₋₄, facultativement substitué par un halogène; un hydroxy, un cyano ou un nitro.

5. Composé selon l'une quelconque des revendications 1-3, dans lequel R₂ est un phényle ou un phényle substitué par un ou plusieurs substituants choisis chacun entre un halogène, un trifluorométhyle, un hydroxy, un alcoxy en C₁₋₄, un nitro, un cyano, un hydroxyalkyle en C₁₋₄ ou un alkyle en C₁₋₄, facultativement substitué par un halogène, un hydroxyle, un cyano ou un nitro.

6. Composé selon la revendication 5, dans lequel ledit substituant est un chloro.

7. Composé selon l'une quelconque des revendications 1-6, dans lequel Y₁ est une liaison et Y₂ est O.

8. Composé selon l'une quelconque des revendications 1-7, dans lequel X₁ est un radical divalent hydrocarboné en C₄₋₁₂ et X₂ est une liaison.

9. Composé selon la revendication 1 lequel est choisi entre le chlorhydrate de chlorure de 1-[2-(4-pipéridyl)éthoxycarbonyloxyméthyl]-4-[*N*'-cyano-*N*"-(6-(4-chlorophénoxy)-1-hexyl)-*N*-guanidino]pyridinium ; le chlorhydrate de chlorure de 1-[3-pipéridylméthoxycarbonyloxyméthyl]-4-[*N*'-cyano-*N*"-(6-(4-chlorophénoxy)-1-hexyl)-*N*-guanidino]pyridinium ; le chlorhydrate de chlorure de 1-[4-pipéridylméthoxycarbonyloxyméthyl]-4-[*N*'-cyano-*N*"-(6-(4-chlorophénoxy)-1-hexyl)-*N*-guanidino]pyridinlum ; le chlorhydrate de chlorure de 1-[4-pipéridylcarbonyloxyméthyl]-4-[N'-cyano-N"-(6-(4-chlorophénoxy)-1-hexyl)-N-guanidino]pyridinium.

10. Chlorhydrate de chlorure de 1-[8-amino-1-octyloxycarbonyloxyméthyl]-4-[N'-cyano-N"-(6-(4-chlorophénoxy)-1-hexyl)-N-guanidino]pyridinium.

11. Composition pharmaceutique comprenant un composé de formule I ou II selon l'une quelconque des revendications 1-10 ainsi qu'un excipient ou diluant acceptable du point de vue pharmaceutique.

12. Composition selon la revendication 11, dans laquelle le composé est dissous dans un solvant acceptable du point de vue pharmaceutique approprié, par exemple choisi entre l'eau, une solution saline isotonique, une solution de glucose isotonique ou une solution tampon.

13. Composition selon la revendication 12 pour une administration parentérale, une injection intraveineuse ou une perfusion.

14. Composition selon l'une quelconque des revendications 11-13 comprenant en plus un ou plusieurs autres composés antinéoplasiques.

15. Composition selon la revendication 14, dans laquelle ledit ou lesdits autres composés antinéoplasiques sont choisis parmi des dérivés de la *S-*triazine, des agents antibiotiques, des agents alkylants, des antimétabolites, des agents antimitotiques, des agents hormonaux, des agents de différenciation, des modificateurs de la réponse biologique et des inhibiteurs de l'angiogenèse.

16. Composition selon la revendication 15, dans laquelle le composé de formule I ou II est le chlorhydrate de chlorure de 1-[2-(4-pipéridyl)éthoxycarbonyloxyméthyl]-4-[N'-cyano-N"-(6-(4-chlorophénoxy)-1-hexyl)-N-guanidino]pyridinium et dans laquelle le ou les autres agents antinéoplasiques sont choisis chacun entre le paclitaxel, le fluorouracile, l'étoposide, le cyclophosphamide, le cisplatine, le carboplatine, la vincristine, la gemcitabine, la vinorelbine, le chlorambucil, la doxorubicine, le melphalan et le seocalcitol.

17. Composition pharmaceutique comprenant, dans des récipients séparés et destinés à une administration simultanée ou successive, un composé de formule I ou II selon l'une quelconque des revendications 1-10 et un ou plusieurs autres composés antinéoplasiques, ainsi que des excipients ou diluants acceptables du point de vue pharmaceutique.

18. Composé selon l'une quelconque des revendications 1-10 destiné à être utilisé comme médicament.

19. Utilisation d'un composé de formule I ou II selon l'une quelconque des revendications 1-10, facultativement en même temps qu'un ou plusieurs autres composés antinéoplasiques, pour la préparation d'un médicament pour le traitement ou l'amélioration de maladies ou affections prolifératives.

20. Utilisation selon la revendication 19, dans laquelle la maladie proliférative est un cancer.

21. Utilisation selon la revendication 19, dans laquelle ladite maladie proliférative est choisie entre la leucémie, la leucémie myéloïde aiguë, la leucémie myéloïde chronique, la leucémie lymphocytaire chronique, la myélodysplasie, le myélome multiple, la maladie de Hodgkin ou un lymphome non Hodgkinien, le cancer du poumon à petites cellules ou à grandes cellules, le cancer de l'estomac, de l'intestin ou colorectal, le cancer de la prostate, des ovaires ou du sein, le cancer de la tête, du cerveau ou du cou, un cancer dans le tractus urinaire, le cancer du rein ou de la vessie, le mélanome malin, le cancer du foie, le cancer de l'utérus ou du pancréas.

22. Utilisation selon l'une quelconque des revendications 19-21, dans laquelle ledit ou lesdits autres composés antinéoplasiques sont choisis parmi des dérivés de la *S*-triazine, des agents antibiotiques, des agents alkylants, des antimétabolites, des agents antimitotiques, des agents hormonaux, des agents de différenciation, des modificateurs de la réponse biologique et des inhibiteurs de l'angiogenèse.

23. Utilisation selon l'une quelconque des revendications 19-22, dans laquelle le composé de formule I ou II est le chlorhydrate de chlorure de 1-[2-(4-pipéridyl)éthoxycarbonyloxyméthyl]-4-[*N'*-cyano-*N"*-(6-(4-chlorophénoxy)-1-hexyl)-*N*-guanidino]pyridinium et dans laquelle ledit autre composé antinéoplasique est choisi entre le paclitaxel, le fluorouracile, l'étoposide, le cyclophosphamide, le cisplatine, le carboplatine, la vincristine, la gemcitabine, la vinorelbine, le chlorambucil, la doxorubicine, le melphalan et le seocalcitol.

24. Composé choisi entre le carbonate de chlorométhyle et de 2-(1-(*tert-*butoxycarbonyl)-4-pipéridyl)éthyle ; le carbonate de chlorométhyle et de 1-(*tert*-butoxycarbonyl)-3-pipéridylméthyle ; le carbonate de chlorométhyle et de 1-(*tert*-butoxycarbonyl)-4-pipéridylméthyle ; le carbonate de iodométhyle et de 2-(1-*(tert*-butoxycarbonyl)-4-pipéridyl)éthyle ; le carbonate de iodométhyle et de 1-(*tert* butoxycarbonyl)-3-pipéridylméthyle ; le carbonate de iodométhyle et de 1-(*tert*-butoxycarbonyl)-4-pipéridylméthyle ; le carbonate de chlorométhyle et de 8-(*tert*-butoxycarbonylamino)-1-octyle ; le carbonate de iodométhyle et de 8-(*tert*-butoxycarbonylamino)-1-octyle ; le *N*-*tert*-butoxycarbonyl-4-pipéridylcarboxylate de chlorométhyle ; le *N*-*tert*-butoxycarbonyl-4-pipéridylcarboxylate de iodométhyle ; l'iodure de 1-[2-[1-(*tert-*butoxycarbonyl)-4-pipéridyl]éthoxycarbonyloxyméthyl]-4-[*N*'-cyano-*N*"-(6-(4-chlorophénoxy)-1-hexyl)-*N*-guanidino]pyridinium ; l'iodure de 1-[1-(*tert*-butoxycarbonyl)-3-pipéridylméthoxycarbonyloxyméthyl]-4-[*N*'-cyano-*N*"-(6-(4-chlorophénoxy)-1-hexyl)-*N*-guanidino]pyridinium ; l'iodure de 1-[1-(*tert*-butoxycarbonyl)-4-pipéridylméthoxycarbonyloxyméthyl]-4-[*N'*-cyano-*N*"-(6-(4-chlorophénoxy)-1-hexyl)-*N*-guanidino]pyridinium ; l'iodure de 1-[8-(*N*-*tert-*butoxycarbonylamino)-1-octyloxycarbonyloxyméthyl]-4-[*N*'-cyano-*N*"-(6-(4-chlorophénoxy)-1-hexyl)-*N*-guanidino]pyridinium et l'iodure de 1-[1-(*tert-*butoxycarbonyl)-4-pipéridylméthoxycarbonyloxyméthyl]-4-[*N*'-cyano-*N*"-(6-(4-chlorophénoxy)-1-hexyl)-*N*-guanidino]pyridinium.

25. Composé selon l'une quelconque des revendications 1-10, facultativement avec un ou plusieurs autres composés antinéoplasiques, destiné à être utilisé dans le traitement ou l'amélioration de maladies ou affections prolifératives.
